(19) Europäisches Patentamt
European Patent Office
Office européen des brevets

(11) EP 4 509 509 A1

(12) **EUROPEAN PATENT APPLICATION**
published in accordance with Art. 153(4) EPC

(43) Date of publication:
**19.02.2025 Bulletin 2025/08**

(21) Application number: **22914356.5**

(22) Date of filing: **19.12.2022**

(51) International Patent Classification (IPC):
***C07D 491/147** (2006.01)* ***C07D 491/048** (2006.01)*

(52) Cooperative Patent Classification (CPC):
Y02P 20/55

(86) International application number:
**PCT/CN2022/139966**

(87) International publication number:
**WO 2023/125102 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.12.2021 CN 202111658903**

(71) Applicant: **Hangzhou HighlightII Pharmaceutical Co., Ltd**
**Hangzhou, Zhejiang 310018 (CN)**

(72) Inventors:
- **LIANG, Congxin**
  **Zhejiang 310012 (CN)**
- **LI, Shuangjiang**
  **Zhejiang 310018 (CN)**
- **HUANG, Junmin**
  **Princeton, Jersey 08540 (US)**
- **SHI, Guoqiang**
  **Zhejiang 311606 (CN)**
- **DUAN, Yaya**
  **Zhejiang 311606 (CN)**
- **YAN, Pucha**
  **Zhejiang 310018 (CN)**

(74) Representative: **Hoffmann Eitle**
**Patent- und Rechtsanwälte PartmbB**
**Arabellastraße 30**
**81925 München (DE)**

# (54) METHOD FOR SYNTHESIZING 1H-FURO[3,2-B]IMIDAZO[4,5-D]PYRIDINE COMPOUND

(57) Provided is a method for synthesizing 1H-furo[3,2-b]imidazo[4,5-d]pyridine compounds. The method comprises the following steps of: reacting compound 1 with compound 2 in a solvent in the presence of a base to obtain compound 3; subjecting the compound 3 to a reduction reaction to obtain compound 4; and subjecting the compound 4 to a ring-closing reaction with compound 5 or compound 5' to obtain compound 6 or a hydrate thereof, wherein R is methyl or ethyl, the methyl or ethyl is optionally substituted by hydroxyl, and X=O or $CH_2$. The method for synthesizing the 1H-furo[3,2-b]imidazo[4,5-d]pyridine compound has the advantages of high yield, fewer impurities and is easy to control, saves the cost. The method is simple and convenient to operate, and is suitable for industrial scale.

EP 4 509 509 A1

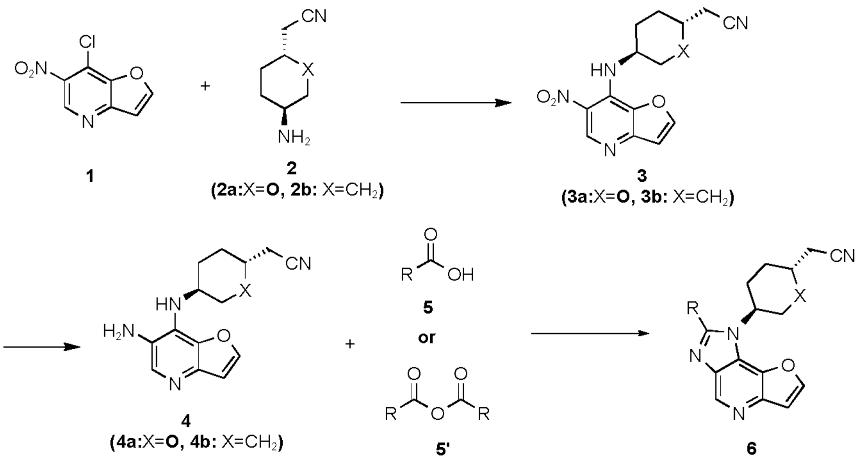

1

2 (2a:X=O, 2b: X=$CH_2$)

3 (3a:X=O, 3b: X=$CH_2$)

4 (4a:X=O, 4b: X=$CH_2$)

5

or

5'

6

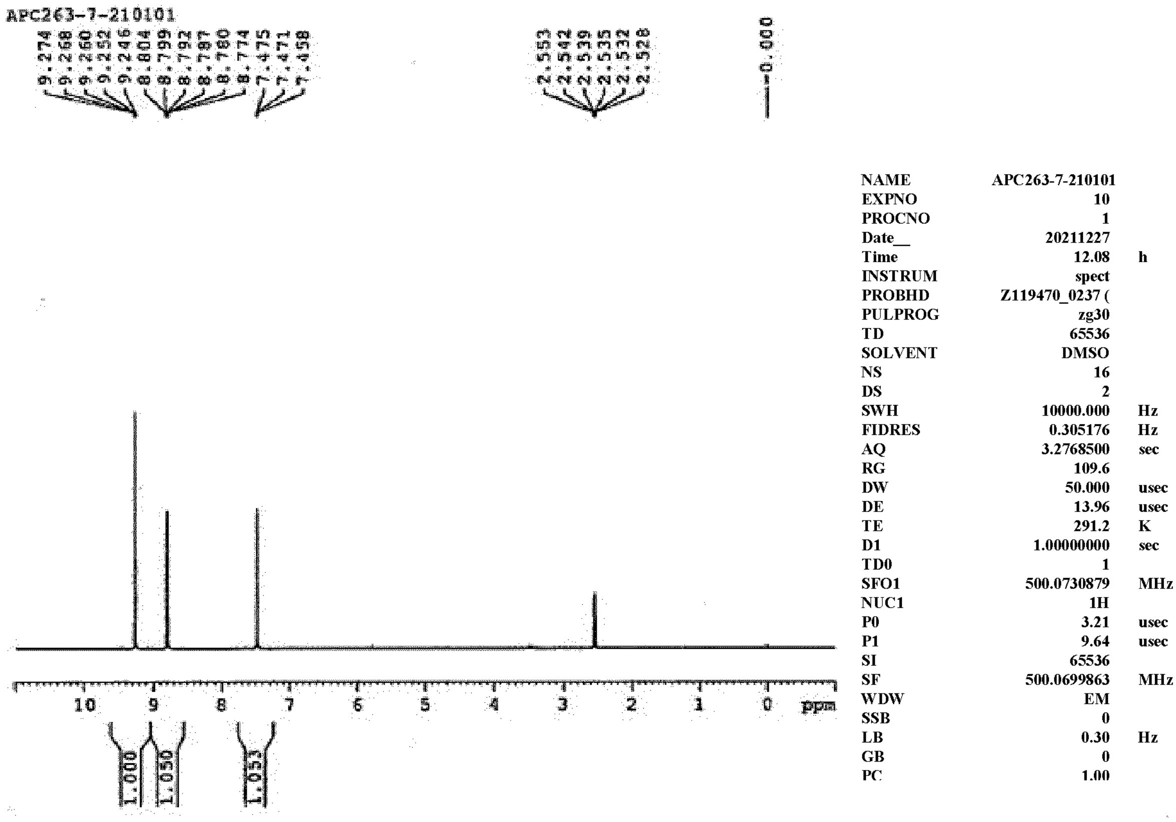


Fig. 1

## Description

### TECHNICAL FIELD

The present invention relates to the field of pharmaceutical synthesis, in particular to methods for synthesizing 1H-furo[3,2-b]imidazo[4,5-d]pyridine compounds.

### BACKGROUND

1H-furo[3,2-b]imidazo[4,5-d]pyridine compounds are highly selective JAK1 and TYK2 inhibitors that selectively inhibit the activity of one or more protein kinases relative to other related kinases. Therefore, they are expected to be used in the treatment of diseases mediated by the selectively inhibited kinase or kinases while avoiding the adverse side effects associated with the inhibitions of other kinases, e.g., for the treatment of disorders associated with the activity of JAK1/TYK2, such as autoimmune diseases or disorders, or inflammatory diseases or disorders, as well as cancers or tumor or disorders.

Methods for synthesizing such compounds in the prior art have the problems of high cost and complex operation, so the methods are not suitable for industrial scale (WO 2018/067422 A1, WO 2020/244348 A1 and WO 2020/244349 A1). In light of this, there is a need to develop a method for synthesizing 1H-furo[3,2-b]imidazo[4,5-d]pyridine compounds, which has the advantages of high yield, fewer impurities, easy control, cost saving, and simple operation, and is thus suitable for industrial scale.

### SUMMARY

In view of the above problems existing in the prior art, the purpose of the present invention is to provide methods for synthesizing 1H-furo[3,2-b]imidazo[4,5-d]pyridine compounds, which save the cost, and are simple and convenient to operate, thus are suitable for industrial scale.

In order to achieve the above purpose, the technical solutions adopted by the present invention are as follows.

A method for synthesizing 1H-furo[3,2-b]imidazo[4,5-d]pyridine compounds (hereinafter referred to as "synthesis route 1", comprises the following steps of:

step 1:

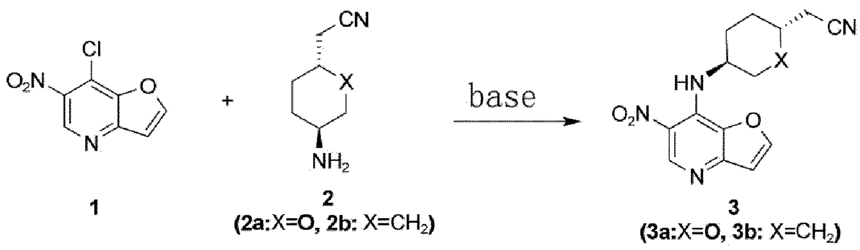


**1** **2** (**2a**:X=**O**, **2b**: X=$CH_2$) **3** (**3a**:X=**O**, **3b**: X=$CH_2$)

reacting compound 1 with compound 2 or compound 2 hydrochloride in a solvent in the presence of a base to obtain compound 3;

step 2:

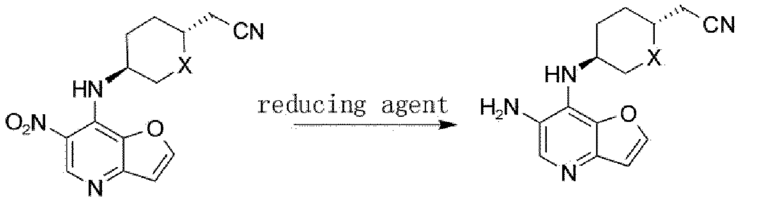


**3** (**3a**:X=**O**, **3b**: X=$CH_2$) **4** (**4a**:X=**O**, **4b**: X=$CH_2$)

subjecting the compound 3 to a reduction reaction to obtain compound 4 or compound 4 hydrochloride; and

step 3:

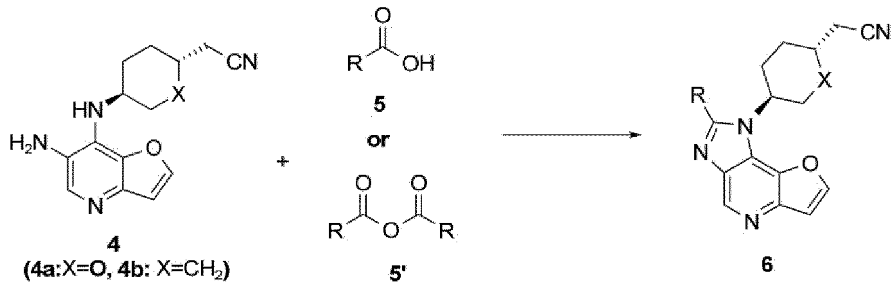

subjecting the compound 4 or compound 4 hydrochloride to a ring-closing reaction with compound 5 or compound 5' to obtain compound 6 or a hydrate of the compound 6, wherein R is methyl or ethyl, the methyl or ethyl is optionally substituted by hydroxyl, and preferably, R is methyl or 1-hydroxyethyl; X=$CH_2$, or O; and the hydrate of the compound 6 is preferably a monohydrate.

According to the embodiments of the present invention, in the step 1,

the base is an organic base other than N,N-diisopropylethylamine, wherein the organic base is preferably 1,8-diazabicyclo[5.4.0]undec-7-ene (DBU); or, the base is an inorganic base, wherein the inorganic base is preferably sodium carbonate ($Na_2CO_3$), potassium bicarbonate ($KHCO_3$) or sodium bicarbonate ($NaHCO_3$), and more preferably sodium bicarbonate ($NaHCO_3$); or, the base is N,N-diisopropylethylamine;

the solvent is ethanol and/or acetonitrile, preferably, the solvent is ethanol, or a mixed solvent of acetonitrile and ethanol, wherein a volume ratio (mL/mL, or L/L) of acetonitrile to ethanol is from 1:0.8 to 1:1.2, and preferably 1:1;

ratio of compound 1 to the compound 2 is from 1:0.8 to 1:1.2, for example, 1:0.8, 1:0.9, 1:1, 1: 1.01, 1: 1.05, 1:1.1, and 1:1.2;

when the compound 2 is in the form of hydrochloride, the molar ratio of compound 1 to the base is from 1:2 to 1:2.5, preferably from 1:2.1 to 1:2.3, more preferably from 1:2.2 to 1:2.25, and most preferably from 1:2.2 to 1:2.23;

when the compound 2 is in a salt-free form, the molar ratio of compound 1 to the base is from 1:1 to 1:1.5, and preferably from 1:1.1 to 1:1.3;

the mass-to-volume ratio (g/mL) of compound 1 to the solvent is from 1:9 to 1:10;

the reaction of the step 1 is optionally carried out under the protection of an inert gas (such as nitrogen);

the reaction is carried out at the temperature of 50-80°C, and lasts for 16-22 hours; and

after the reaction is completed, the reaction solution is cooled, stirred and filtered, and the filter cake is washed and dried to obtain the compound 3 (for example, after the reaction is completed, the reaction solution is cooled to 20-30°C, water is added to the solution and the solution is stirred at 20-30°C for 0.5-2 hours, then cooled to 0-15°C and stirred at 0-15°C for 2-4 hours, then filtered, and the filter cake is washed and dried to obtain the compound 3).

According to the embodiments of the present invention, in the step 2,

the reducing agent used in the reduction reaction is a nitro-reducing agent other than $H_2$/Pd;

the reducing agent used in the reduction reaction is Fe/acetic acid, or $B_2(OH)_4$, or $SnCl_2$;

when the reducing agent is Fe/acetic acid, the solvent used in the reduction reaction is acetonitrile; the molar ratio of compound 3 to Fe is from 1:6 to 1:9, and the molar ratio of compound 3 to the acetic acid is from 1:12 to 1:14; and a mass-to-volume ratio (g/mL) of the compound 3 to the solvent is from 1:4 to 1:10, and preferably from 1:5 to 1:9; and

when the reducing agent is $B_2(OH)_4$, the solvent used in the reduction reaction is a mixed solvent of water and methanol, wherein a volume ratio (mL/mL, or L/L) of water to methanol is from 1:1 to 9:1, preferably from 3:1 to 5:1, more preferably 4:1; the molar ratio of compound 3 to $B_2(OH)_4$ is from 1:3 to 1:5, and preferably 1:3.5; a mass-to-volume ratio (g/mL) of the compound 3 to the solvent is from 1:4 to 1:10, and preferably from 1:5 to 1:9; and $B_2(OH)_4$ is added portionwise;

when the reducing agent is $SnCl_2$, the solvent used in the reduction reaction is ethyl acetate, wherein the molar ratio of compound 3 to $SnCl_2$ is from 1:2 to 1:9, and preferably from 1:3 to 1:6; and a mass-to-volume ratio (g/mL) of the compound 3 to the solvent is from 1:4 to 1:20, and preferably from 1:5 to 1:15; and

when the reducing agent is $B_2(OH)_4$, 4,4'-bipyridine is optionally added;

the reaction of the step 2 is optionally carried out under the protection of inert gas (such as nitrogen);

the reaction is carried out at a temperature of 15-90°C, and lasts for 1-18 hours;

when the reducing agent is Fe/acetic acid, after the reaction is completed, the reaction solution is filtered before cooling down, and rinsed with hot acetonitrile. The pH is adjusted to about 6 (for example, the pH is adjusted to 6) with sodium citrate solution, and then the pH is adjusted to 8-9 with $K_3PO_4$ solution (for example, 30% $K_3PO_4$ solution). After concentration, celite, water and ethyl acetate are added to the reaction solution, and the solution is filtered and layered. The obtained aqueous phase is extracted with ethyl acetate and the obtained organic phases are combined and washed with sodium citrate aqueous solution and brine respectively. Silica gel and anhydrous sodium sulfate are added, and the solution is filtered, washed with ethyl acetate, concentrated. Methyl tert-butyl ether is added, and the precipitate is filtered and dried to obtain the compound 4;

when the reducing agent is $B_2(OH)_4$, after the reaction is completed, the temperature is reduced to 35-45°C. Part of the solvent is concentrated under reduced pressure, then the temperature is reduced to 0-10°C and the reaction solution is stirred and filtered. The filter cake is rinsed with dichloromethane and the filtrate is collected. Sodium bicarbonate is added portionwise and stirred at 20-30°C. The system is allowed to settle, then the organic phase is separated and the aqueous phase is extracted with dichloromethane. The obtained organic phases are combined, dried with anhydrous sodium sulfate. Hydrogen chloride in ethanol solution is dropwise added at 15-25°C and stirred with the temperature kept at 15-25°C. Ethyl acetate is dropwise added at 15-25°C and stirred with the temperature kept at 15-25°C. The mixture is filtered, then the filter cake is rinsed with ethyl acetate, and dried to obtain compound 4 hydrochloride; and

when the reducing agent is $SnCl_2$, after the reaction is completed, the temperature is reduced to room temperature. Sodium bicarbonate is added with an ice bath, then the reaction solution is stirred and filtered. The filter cake is rinsed with ethyl acetate, and the filtrate is collected and washed for three times with aqueous solution of saturated sodium bicarbonate. The obtained organic phases are combined and washed for three times with brine, then the organic phases are combined, dried with anhydrous sodium sulfate, filtered, and the filtrate is concentrated under reduced pressure to obtain the compound 4.

According to the embodiments of the present invention, in the step 3,

the compound 5 is R-lactic acid

( 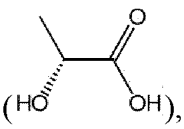 ),

and the compound 5' is acetic acid or acetic anhydride;

the ring-closing reaction is carried out in a solvent, wherein the solvent is toluene, dioxane, acetic acid, methylcyclohexane or acetic anhydride;

when the compound 4 is subjected to the ring-closing reaction with R-lactic acid, the molar ratio of compound 4 to the R-lactic acid is from 1:4 to 1:30, preferably from 1:4 to 1:20, and more preferably from 1:4 to 1:10; and a mass-to-volume ratio (g/mL) of the compound 4 to the solvent is from 1:1.5 to 1:40, preferably from 1:1.5 to 1:30, and more preferably from 1:1.5 to 1:15;

when the compound 4 is subjected to the ring-closing reaction with acetic anhydride or acetic acid, the acetic

anhydride or the acetic acid act as a reaction reagent and a solvent simultaneously, and the mass-to-volume ratio (g/mL) of the compound 4 to the acetic anhydride or the acetic acid is from 1:1 to 1:10, and preferably from 1:2 to 1:5;

an acid catalyst is optionally added in the ring-closing reaction of the compound 4 with the compound 5 or the compound 5', wherein the acid catalyst is preferably methanesulfonic acid;

in the ring-closing reaction between the compound 4 and the compound 5', when the reacting reagent/solvent is acetic anhydride, acetic acid (AcOH) or sodium acetate (NaOAc) is optionally added;

in the ring-closing reaction between the compound 4 and the compound 5', when the reacting reagent/solvent is acetic acid, sodium acetate (NaOAc) is optionally added;

the reaction is carried out at a temperature of 80-120°C, and lasts for 5-24 hours, and preferably 16-18 hours; and

after the reaction is completed, the reaction solution is cooled and extracted, the pH is adjusted to 7-10, and the obtained solid is dried to obtain the compound 6 or the hydrate of the compound 6.

According to the embodiments of the present invention, the present invention provides a method for synthesizing 1H-furo[3,2-b]imidazo[4,5-d]pyridine compound (using "synthesis route 1", used for synthesizing compound 6A, 6C or a hydrate thereof, preferably a monohydrate), comprising the following steps of:

step 1:

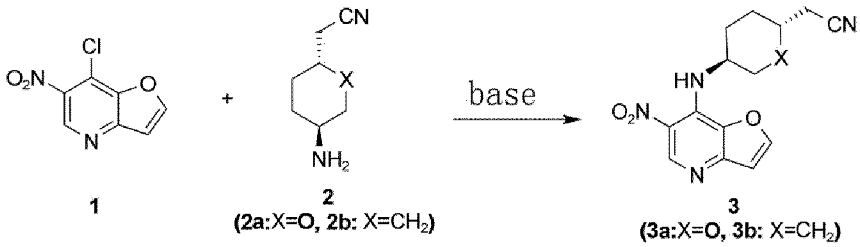


1 2 (**2a**:X=O, **2b**: X=$CH_2$) 3 (**3a**:X=O, **3b**: X=$CH_2$)

reacting compound 1 with compound 2 or compound 2 hydrochloride in a solvent in the presence of a base to obtain compound 3;

step 2:

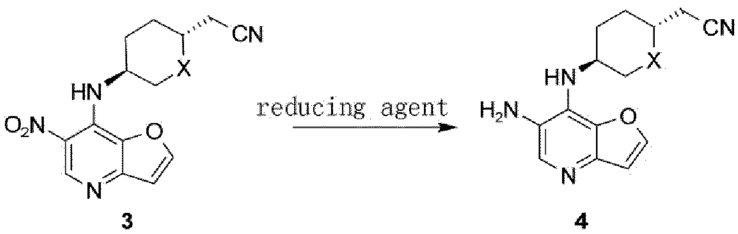


3 (**3a**:X=O, **3b**: X=$CH_2$) 4 (**4a**:X=O, **4b**: X=$CH_2$)

subjecting the compound 3 to a reduction reaction to obtain compound 4 or compound 4 hydrochloride; and

step 3:

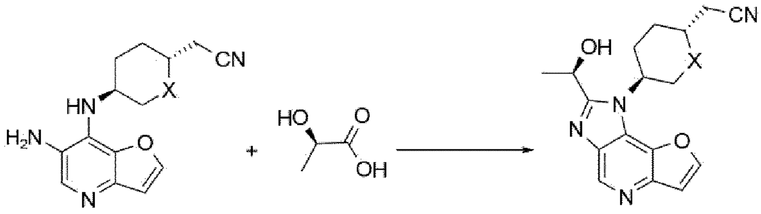

**4**
**(4a:**X=**O, 4b:** X=$CH_2$**)**

**6A(X=O),**
**6C(X=$CH_2$)**

subjecting the compound 4 or compound 4 hydrochloride to a ring-closing reaction with the **R-**lactic acid to obtain the compounds 6A and 6C, or the hydrates thereof.

According to the embodiments of the present invention, in the method for synthesizing compounds 6A and 6C or the hydrates thereof, the step 1 and the step 2 are as described above, and in the step 3,

the ring-closing reaction is carried out in a solvent, wherein the solvent is toluene, dioxane, or methylcyclohexane, preferably toluene or methylcyclohexane, and more preferably methylcyclohexane;

the molar ratio of compound 4 to R-lactic acid is from 1:4 to 1:30, preferably from 1:4 to 1:20, more preferably from 1:4 to 1:10, and most preferably from 1:5 to 1:7;

a mass-to-volume ratio (g/mL) of the compound 4 to the solvent is from 1:1.5 to 1:40, preferably from 1:1.5 to 1:30, more preferably from 1:1.5 to 1:15, and most preferably from 1:1.5 to 1:5;

the reaction is refluxed with a Dean-Stark apparatus at 87-110°C for 5-24 hours; and

after the reaction is completed, methanol is added and the methanol layer is separated. Sodium hydroxide solution is added with stirring. Hydrochloric acid is dropwise added to adjust the pH to about 7. The solvent is removed under reduced pressure, and water is added with stirring. The solid is filtered, washed with water, and dried to obtain the compound 6A or 6C (for example, in the process of synthesizing the compound 6A, after the reaction is completed, methanol is added, the reaction solution is cooled to 20-30°C, methanol is added (for example, 3 times the volume), and the resulting mixture is stirred and layered to obtain a methanol layer. The methanol layer is cooled to -10 - 0°C, added sodium hydroxide solution, stirred, and dropwise added hydrochloric acid to adjust pH to 7, then the solvent is removed under reduced pressure, water is added and the obtained system is stirred, filtered, washed with water, and dried to obtain the compound 6A).

According to the embodiments of the present invention, the present invention provides a method for synthesizing 1H-furo[3,2-b]imidazo[4,5-d]pyridine compound (using "synthesis route 1") for synthesizing compound 6B or 6D, comprising the following steps of:

step 1:

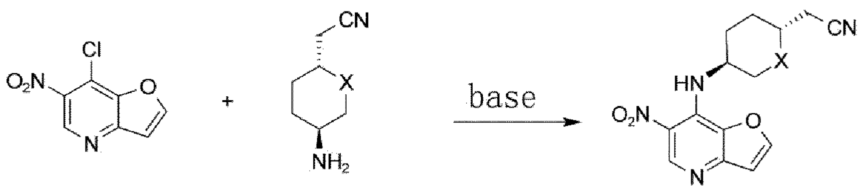


**1**

**2**
**(2a:**X=**O, 2b:** X=$CH_2$**)**

**3**
**(3a:**X=**O, 3b:** X=$CH_2$**)**

reacting compound 1 with compound 2 or compound 2 hydrochloride in a solvent in the presence of a base to obtain compound 3;

step 2:

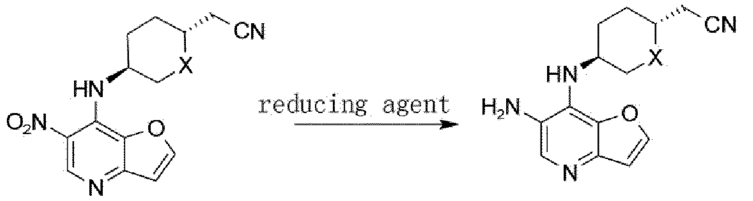


**3**
**(3a:**X=**O, 3b:** X=$CH_2$**)**

**4**
**(4a:**X=**O, 4b:** X=$CH_2$**)**

subjecting the compound 3 to a reduction reaction to obtain compound 4 or compound 4 hydrochloride; and

step 3:

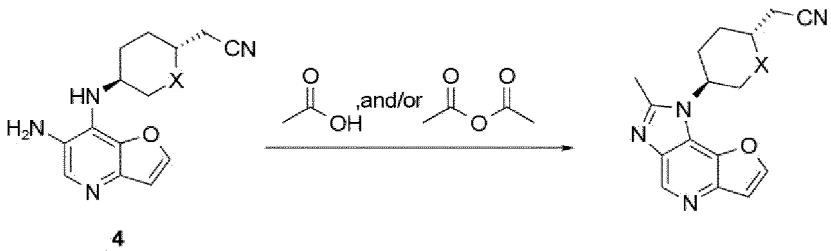


**4**
**(4a:**X=**O, 4b:** X=$CH_2$**)**

**6B(X=O);**
**6D(X=$CH_2$)**

subjecting the compound 4 or compound 4 hydrochloride to a ring-closing reaction with the acetic anhydride and/or acetic acid to obtain the compound 6B or the compound 6D.

According to the embodiments of the present invention, in the method for synthesizing compound 6B or 6D, the step 1 and the step 2 are as described above, and in the step 3,

when the compound 4 is subjected to the ring-closing reaction with acetic anhydride, acetic acid or sodium acetate is optionally added, wherein the molar ratio of compound 4 to acetic acid is from 1:0.1 to 1:0.5, and preferably from 1:0.2 to 1:0.3, for example, 1:0.2; and the molar ratio of compound 4 to sodium acetate is from 1:0.2 to 1:1, and preferably from 1:0.4 to 1:0.8, for example, 1:0.5;

when the compound 4 is subjected to the ring-closing reaction with acetic acid, sodium acetate is optionally added, wherein the molar ratio of compound 4 to sodium acetate is from 1:1 to 1:3, and preferably from 1:1.5 to 1:2.5, for example, 1:2;

when the compound 4 is subjected to the ring-closing reaction with acetic anhydride, the mass-to-volume ratio of compound 4 to acetic anhydride is from 1:2 to 1:5, and preferably 1:3;

when the compound 4 is subjected to the ring-closing reaction with acetic acid, the mass-to-volume ratio of compound 4 to acetic acid is from 1:3 to 1:8, and preferably 1:5;

the reaction is carried out at a temperature of 80-120°C, and preferably 90-110°C, and lasts for 12-24 hours, and preferably 12-18 hours; and

after the reaction is completed, methyl tert-butyl ether is added to the reaction solution, and the obtained system is stirred evenly, and filtered. The filter cake is dissolved in a mixed solvent of water and dichloromethane, and NaOH aqueous solution is dropwise added to adjust pH to 7-10. The resulting solution is layered and separated. Then the obtained aqueous layer is extracted with dichloromethane, and the obtained organic phases are combined, dried with anhydrous $Na_2SO_4$, filtered, and concentrated. To the system was added ethanol, and water with stirring. The obtained system is filtered, then the filter cake is dried to obtain the compound 6B or 6D (for example, in the reaction of synthesizing the compound 6B, after the reaction is completed, the reaction solution is added with methyl tert-butyl ether, stirred evenly at 0-10°C, and filtered, the filter cake is dissolved in the mixed solvent of water and dichloromethane, 10% NaOH aqueous solution is dropwise added to adjust the pH to 7-10. The resulting solution is layered and separated. Then the obtained aqueous layer is extracted with dichloromethane, and the obtained organic phases

are combined, dried with anhydrous $Na_2SO_4$, filtered, concentrated. Ethanol is added and the obtained system becomes clear at 45-55°C. The solution is cooled to 0-10°C for precipitation, then water is added with stirring. The mixture is filtered, and the filter cake is dried to obtain the compound 6B).

According to the embodiments of the present invention, the present invention further provides a method for synthesizing compound 1, comprising the following steps of:

step 1-1:

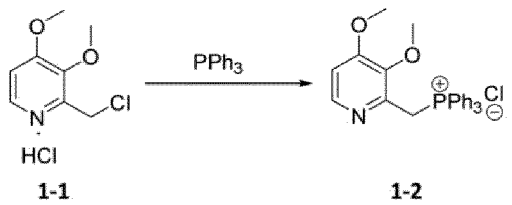


reacting compound 1-1 with triphenylphosphine to obtain compound 1-2;

step 1-2:

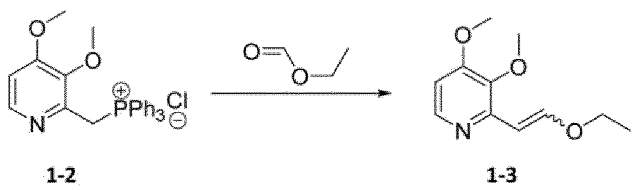

reacting the compound 1-2 with ethyl formate to obtain compound 1-3;

step 1-3:

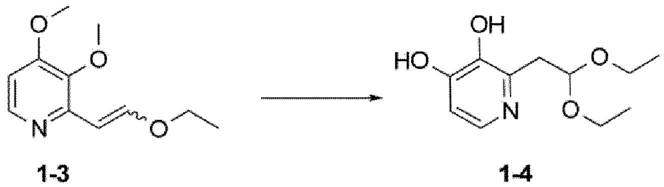

subjecting the compound 1-3 to a demethylation and acetalization reaction to obtain compound 1-4;

step 1-4:

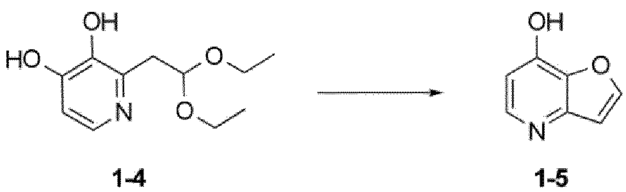

subjecting the compound 1-4 to a ring-closing reaction under acidic condition to obtain compound 1-5;

step 1-5:

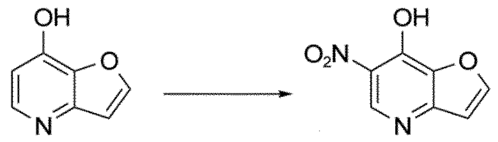

1-5 1-6

subjecting the compound 1-5 to a nitration reaction to obtain compound 1-6; and

step 1-6:

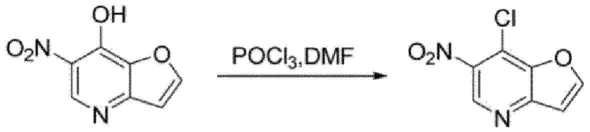


1-6 1

subjecting the compound 1-6 to a chloro-substitution reaction to obtain the compound 1.

According to the embodiments of the present invention, in the method for synthesizing the compound 1, preferably,

in the step 1-1:

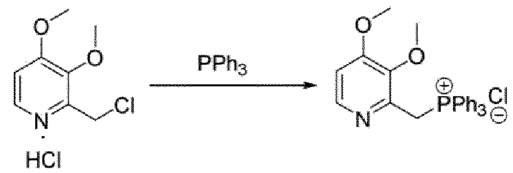


1-1 1-2

sodium carbonate is added to a mixture of compound 1-1, toluene and water in batches. The resulting mixture is stirred under room temperature, and layered. The obtained aqueous phase is extracted with toluene, and the obtained organic phases are combined, washed with brine, dried with anhydrous sodium sulfate, and filtered. Triphenylphosphine is added to the filtrate, and the obtained system is heated to 110-120°C (preferably 108-115°C) with a Dean-Stark apparatus for 14-20 hours (preferably 16-18 hours). The reaction is cooled to 20-30°C, filtered and rinsed with toluene, and then the wet product is dried under reduced pressure to obtain the compound 1-2;

in the step 1-2:

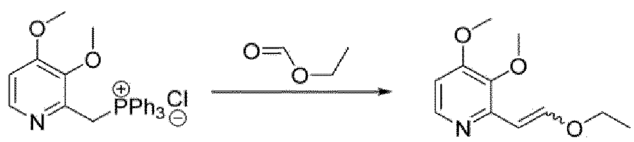

1-2 1-3

the mixture of compound 1-2 and dimethyl sulphoxide is cooled to 18-25°C, an alkali metal tert-butoxide is added in batches and the temperature is controlled at 20-35°C, then the reaction solution is stirred at room temperature for 1-2 hours after the addition. Ethyl formate is dropwise added, and the obtained system is stirred at 40-50°C for 8-12h before cooling to room temperature. Organic solvent and water are added, and the obtained system is extracted and layered. The obtained organic phase is washed with water, adjusted pH to 5-6 with hydrochloric acid, and layered. The obtained aqueous phase is extracted with an organic solvent, adjusted pH is to be greater than or equal to 8 with a base, and layered. The obtained aqueous phase is extracted with organic solvent, and the obtained organic phases are combined, washed with water, dried with anhydrous sodium sulfate, filtered, and concentrated to obtain the compound 1-3;

in the step 1-3:

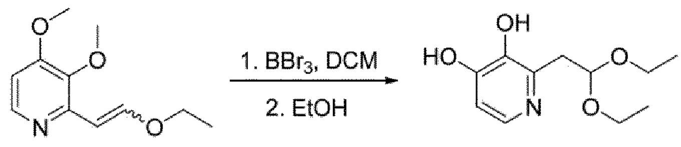


1-3 1-4

a mixture of the compound 1-3 and dichloromethane is cooled to 10-20°C, and boron tribromide is dropwise added at 20-30°C. The reaction solution is stirred for 1-1.5 hours at 20-30°C before quenched by dropwise adding the reaction solution into ethanol at 10-35°C and the resulting mixture is stirred for 0.5-2.5 hours. The solvent is removed under reduced pressure, then ethanol is added and the solvent is removed under reduced pressure. The system is cooled to 15-20°C, and an ethanol solution of sodium ethoxide is dropwise added. The temperature is controlled at 15-30°C, and the dropwise addition is stopped until the pH is 5-6. The reaction solution is filtered, and rinsed with ethanol. To the filtrate is dropwise added an ethanol solution of sodium ethoxide at 15-30°C, and the dropwise addition is stopped until the pH of the material is 8-9. The solvent is removed under reduced pressure, and methyl tert-butyl ether is added. The obtained system is cooled to 5-15°C, stirred for 0.5-1 hour, and filtered. The filter cake is rinsed with methyl tert-butyl ether, and dried to obtain the compound 1-4;

in the step 1-4:

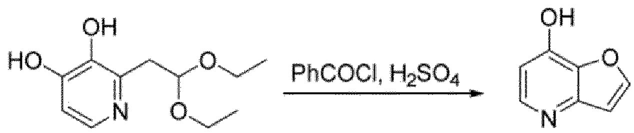


1-4 1-5

benzoyl chloride is dropwise added to a dichloromethane solution of the compound 1-4 at 0-10°C. The obtained system is stirred for 1-1.5 hours at 0-10°C. The reaction solution is dropwise added into concentrated sulfuric acid at 15-25°C, and stirred for 0.5-1.5 hours at 15-25°C. The reaction solution is dropwise added into water, and the temperature is controlled to be not higher than 25°C during the dropwise addition. The resulting mixture is filtered, and the filtrate is dropwise added into aqueous ammonia (the pH is adjusted to 7-8), stirred for 0.5-1 hour, and filtered. The filter cake is rinsed with water, and dried to obtain the compound 1-5;

in the step 1-5:

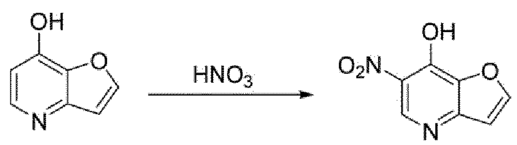


1-5 1-6

Nitric acid is dropwise added to a mixture of acetic acid and acetic anhydride, the temperature is controlled at 15-30°C during the dropwise addition. The reaction solution is stirred evenly at 20-30°C after the dropwise addition. The mixed solution of acetic acid, acetic anhydride and nitric acid is dropwise added into an acetic acid solution of the compound 1-5. The temperature is controlled at 110-120°C during the dropwise addition. The reaction solution is stirred for 1-1.5 hours at 110-120°C. The reaction solution is cooled to 20-30°C, and methyl tert-butyl ether is added. The obtained system is stirred evenly, and filtered. The filter cake is rinsed with methyl tert-butyl ether, and dried to obtain the compound 1-6; and

in the step 1-6:

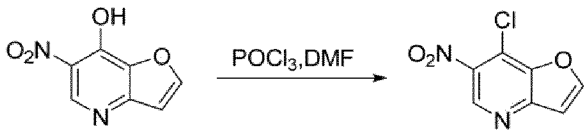


1-6 1

phosphorus oxychloride is dropwise added to a mixture of the compound 1-6, N,N-dimethylformamide and toluene, and the resulting mixture is stirred at 35-75°C for 1-2 hours. Then the reaction solution is cooled to room temperature and toluene is removed. Dichloromethane and 10% potassium carbonate aqueous solution or 5% sodium bicarbonate aqueous solution are added, and the obtained system is allowed to be layered. The obtained organic phases are washed with brine, dried with anhydrous sodium sulfate, filtered, and the solvent is removed to obtain the compound 1.

According to the embodiments of the present invention, further, in the step 1-1,

in a mixture of the compound 1-1, toluene and water, a mass-to-volume ratio (g/mL) of the compound 1-1 to toluene and water is from 1:5 to 1:6, and a volume ratio of toluene to water is from 8:1 to 9:1;

the molar ratio of compound 1-1 to the sodium carbonate is from 1:1.1 to 1:1.2;

the molar ratio of compound 1-1 to the triphenylphosphine is from 1:1.1 to 1:1.2; and

the reaction of the step 1-1 is optionally carried out under the protection of an inert gas (such as nitrogen).

According to the embodiments of the present invention, further, in the step 1-2,

the alkali metal tert-butoxide is potassium tert-butoxide or sodium tert-butoxide;

the base used for adjusting the pH is aqueous ammonia or sodium carbonate;

the organic solvent used for the extraction is methyl tert-butyl ether or ethyl acetate;

the mass-to-volume ratio of compound 1-2 to dimethyl sulphoxide is from 1:3 to 1:5;

the molar ratio of compound 1-2 to the alkali metal tert-butoxide is from 1:1.05 to 1:1.15, and preferably 1:1.1;

the molar ratio of compound 1-2 to ethyl formate is from 1:4 to 1:5; and

the reaction of the step 1-2 is optionally carried out under the protection of an inert gas (such as nitrogen).

According to the embodiments of the present invention, further, in the step 1-3,
**[0019]** A mass-to-volume ratio (g/mL) of the compound 1-3 to the dichloromethane is from 1:6 to 1:10;

the molar ratio of compound 1-3 to the boron tribromide is from 1:2.0 to 1:3.5, and preferably from 1:2.5 to 1:3.2; and the reaction of the step 1-3 is optionally carried out under the protection of an inert gas (such as nitrogen).

According to the embodiments of the present invention, further, in the step 1-4,

a mass-to-volume ratio (g/mL) of the compound 1-4 to the concentrated sulfuric acid is from 1:2 to 1:5;

a mass-to-volume ratio (g/mL) of the compound 1-4 to dichloromethane is from 1:25 to 1:26; and

the molar ratio of compound 1-4 to the benzoyl chloride is from 1:1.5 to 1:2.5, and preferably 1:2.

According to the embodiments of the present invention, further, in the step 1-5,

in the acetic acid solution of the compound 1-5, the mass ratio of compound 1-5 to the acetic acid is from 1:9 to 1:11, and preferably 1:10;

the mass ratio of compound 1-5 to the acetic acid in the mixed solution of acetic acid, acetic anhydride and nitric acid is from 1:3 to 1:5;

the molar ratio of compound 1-5 to the acetic anhydride is from 1:2 to 1:5, and preferably from 1:2.6 to 1:4.2;

the molar ratio of compound 1-5 to the nitric acid is from 1:2 to 1:5, and preferably from 1:2.5 to 1:4.0; and

the reaction of the step 1-5 is optionally carried out under the protection of an inert gas (such as nitrogen).

According to the embodiments of the present invention, further, in the step 1-6,

the molar ratio of compound 1-6 to N,N-dimethylformamide is from 1:1 to 1:1.2;

the mass-to-volume ratio of compound 1-6 to toluene is from 1:9 to 1:11, and preferably 1:10;

the molar ratio of compound 1-6 to phosphorus oxychloride is from 1:1.8 to 1:2.2, and preferably 1:2; and

the reaction of the step 1-6 is optionally carried out under the protection of an inert gas (such as nitrogen).

According to the embodiments of the present invention, when the compound 2 is compound 2a, the present invention further provides a method for synthesizing compound 2a hydrochloride, comprising the following steps of:

step 2-1:

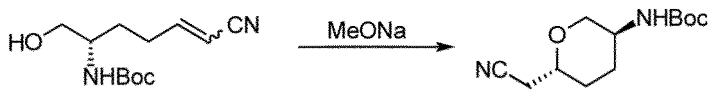


**2-1** **2-2**

subjecting compound 2-1 to a ring-closing reaction under the catalysis of sodium methoxide to obtain compound 2-2; and
step 2-2:

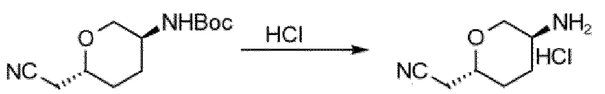


**2-2** **2a**

reacting the compound 2-2 with hydrochloric acid to obtain the compound 2a hydrochloride.

According to the embodiments of the present invention, in the method for synthesizing the N, N compound 2a hydrochloride, preferably,

in the step 2-1:

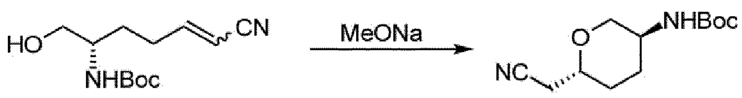


**2-1** **2-2**

a methanol solution of sodium methoxide is dropwise added to a mixture of compound 2-1 and tetrahydrofuran, and the temperature of the system is controlled at 0-10°C during the dropwise addition. The system is stirred at 20-30°C for 3-8 hours. After the reaction is completed, an ammonium chloride solution is dropwise added into the reaction solution at 15-25°C to adjust the pH to 7-8. The resulting mixture is extracted with methyl tert-butyl ether, washed with sodium chloride solution, dried with anhydrous sodium sulfate, cooled to 0-10°C, and filtered, and then the filter cake is dried to

obtain the compound 2-2; and

in the step 2-2:

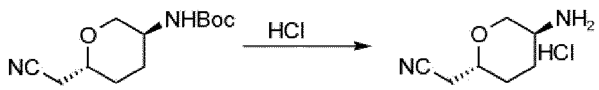


2-2 2a

an ethanol solution of hydrogen chloride is dropwise added to a mixture of the compound 2-2 and dichloromethane. The reaction is stirred at 20-25°C for 4-8 hours. The reaction mixture is cooled to 0-5°C, and filtered, and then the filter cake is rinsed with dichloromethane, and dried to obtain the compound 2a hydrochloride.

According to the embodiments of the present invention, further, in the step 2-1,

the mass-to-volume ratio (g/mL) of compound 2-1 to tetrahydrofuran is from 1:10 to 1:12, and preferably from 1:10 to 1:11;

the molar ratio of compound 2-1 to sodium methoxide in methanol solution is from 1:0.2 to 1:0.3, and preferably 1:0.25; and

the reaction of the step 2-1 is optionally carried out under the protection of an inert gas (such as nitrogen).

According to the embodiments of the present invention, further, in the step 2-2,

the mass-to-volume ratio of compound 2-2 to dichloromethane is from 1:5 to 1:7, and preferably 1:6;

the molar ratio of compound 2-2 to hydrogen chloride in ethanol solution is from 1:2 to 1:5, and preferably 1:3; and

the reaction of the step 2-2 is optionally carried out under the protection of an inert gas (such as nitrogen).

According to the embodiments of the present invention, the present invention provides another method for synthesizing a 1H-furo[3,2-b]imidazo[4,5-d]pyridine compound (hereinafter referred to as "synthesis route 2" for synthesizing compounds 6A and 6B), comprising the following steps of:

step 1A:

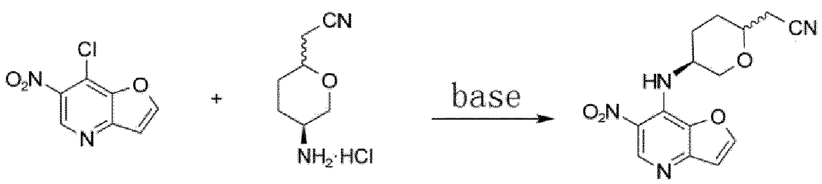


1 7 8

reacting compound 1 with cis-trans isomer mixture 7 in a solvent in the presence of a base to obtain cis-trans isomer mixture 8;

step 2A:

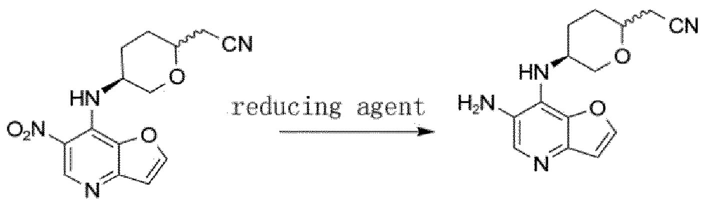


8 9

subjecting the cis-trans isomer mixture 8 to a reduction reaction to obtain cis-trans isomer mixture 9 or the mixture 9 hydrochlorides;

step 3A:

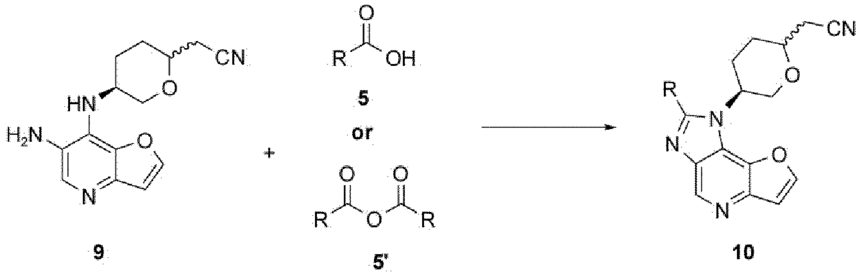

subjecting the cis-trans isomer mixture 9 or the mixture 9 hydrochlorides to a ring-closing reaction with compound 5 or compound 5' to obtain a mixture 10 or hydrates of the mixture 10, wherein R is methyl or ethyl, the methyl or ethyl is optionally substituted by hydroxyl, and preferably, R is methyl or 1-hydroxyethyl, and the hydrates of the mixture 10 is preferably monohydrates of the mixture 10; and

step 4A:

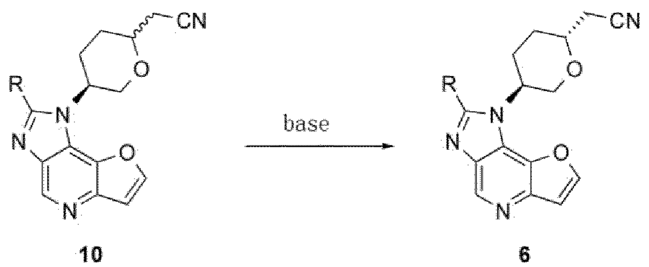


subjecting the mixture 10 or the hydrates of the mixture 10 to an isomer transformation reaction under basic condition to obtain compound 6 or a hydrate of the compound 6, wherein the hydrate of compound 6 is preferably a monohydrate of compound 6.

According to the embodiments of the present invention, in the synthesis route 2, the steps 1A, 2A and 3A are in accordance with the steps 1, 2 and 3 of the synthesis route 1. In the step 4A, the reaction is carried out under basic condition, and the base is alkoxide base, preferably alkali metal $C_{1-6}$ alkoxide, more preferably, potassium tert-butoxide.

According to the embodiments of the present invention, the present invention provides another method for synthesizing the compound 6A, comprising step 1A, step 2A, step 3A and step 4A, wherein the steps 1A, 2A and 3A are in accordance with the methods described in the steps 1, 2 and 3 for preparing the compound 6A (synthesis route 1) above, wherein:

step 1A:

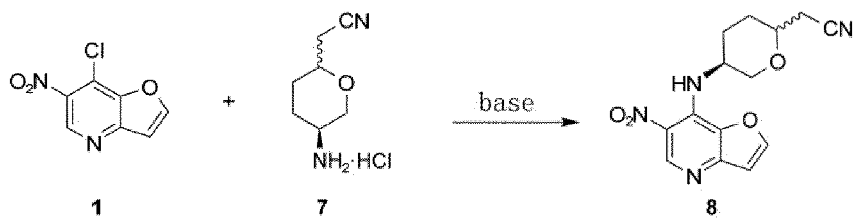


reacting compound 1 with cis-trans isomer mixture 7 in a solvent in the presence of a base to obtain cis-trans isomer mixture 8;

step 2A:

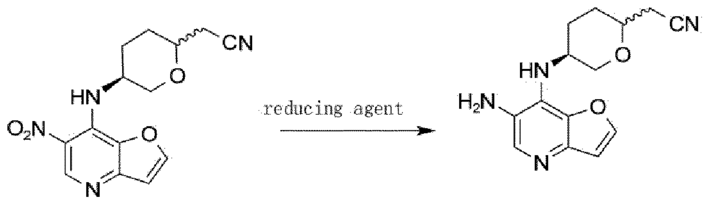


8 9

subjecting the cis-trans isomer mixture 8 to a reduction reaction to obtain cis-trans isomer mixture 9 or the mixture 9 hydrochlorides;

step 3A:

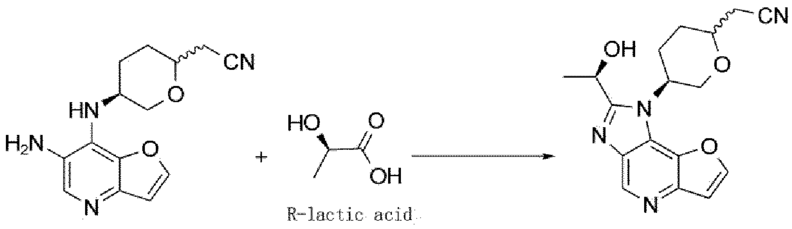


9 6Aa

subjecting the cis-trans isomer mixture 9 or the mixture 9 hydrochlorides to a ring-closing reaction with R-lactic acid to obtain mixture 6Aa or hydrates of the mixture 6Aa; and

step 4A:

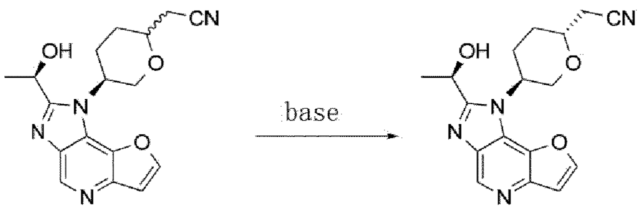


6Aa 6A

subjecting the mixture 6Aa or the hydrates of the mixture 6Aa to an isomer transformation reaction under basic condition to obtain a hydrate of compound 6A. The base is alkoxide base, preferably alkali metal $C_{1-6}$ alkoxide, and more preferably, potassium tert-butoxide; and the hydrate of the compound 6A is preferably a monohydrate of compound 6A.

According to the embodiments of the present invention, in the method for synthesizing the compound 6A through steps 1A to 4A, in the step 4A,

the reaction is carried out in a solvent, wherein the solvent is preferably tetrahydrofuran;

the molar ratio of mixture 6Aa to the base is from 1:0.05 to 1:0.2, and preferably from 1:0.1 to 1:0.2;

the reaction is carried out at room temperature; and

after the reaction is completed, dilute hydrochloric acid is added to adjust the pH to 6-7, the solvent is removed by rotary evaporation. Water is added to the reaction solution, the obtained system is stirred, filtered, and washed with water. The filter cake is collected and dried at 50°C to obtain the hydrate of the compound 6A.

According to the embodiments of the present invention, the present invention provides another method for synthesizing the compound 6B, comprising step 1A, step 2A, step 3A and step 4A, wherein the steps 1A, 2A and 3A are in accordance with the methods described in the steps 1, 2 and 3 for preparing the compound 6B (synthesis route 1) above,

wherein:

step 1A:

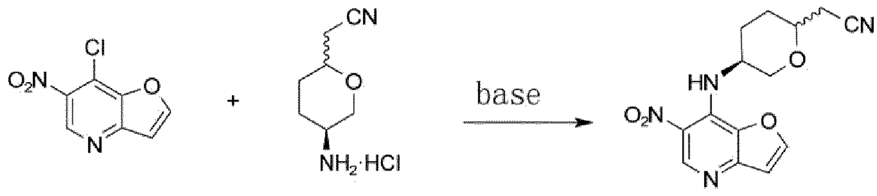


1 7 8

reacting compound 1 with cis-trans isomer mixture 7 in a solvent in the presence of a base to obtain cis-trans isomer mixture 8;

step 2A:

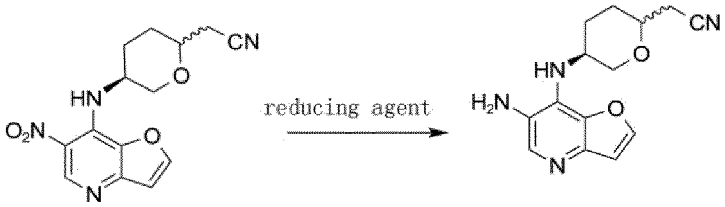


8 9

subjecting the cis-trans isomer mixture 8 to a reduction reaction to obtain cis-trans isomer mixture 9 or the mixture 9 hydrochlorides;

step 3A:

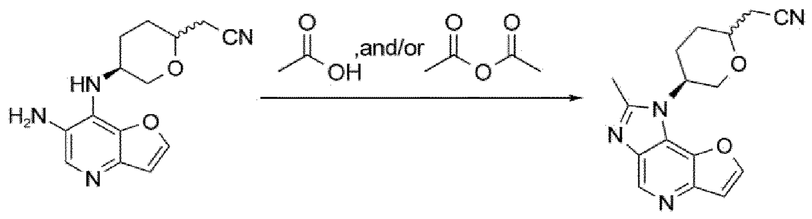


9 6Ba

subjecting the cis-trans isomer mixture 9 or the mixture 9 hydrochlorides to a ring-closing reaction with acetic anhydride and/or acetic acid to obtain mixture 6Ba; and

step 4A:

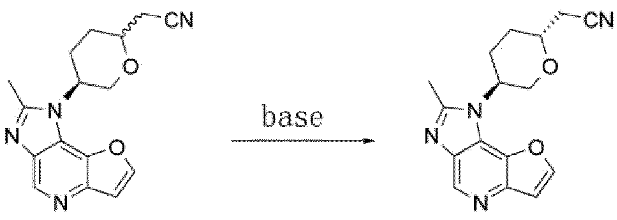


6Ba 6B

the mixture 6Ba is subjected to an isomer transformation reaction under basic condition to obtain the compound 6B. The base is alkoxide base, preferably alkali metal $C_{1-6}$ alkoxide, and more preferably, potassium tert-butoxide.

According to the embodiments of the present invention, in the method for synthesizing compound 6B through

steps 1A to 4A, in step 4A,

the reaction is carried out in a solvent, wherein the solvent is preferably tetrahydrofuran;

the molar ratio of mixture 6Ba to the base is from 1:0.05 to 1:0.2, and preferably from 1:0.1 to 1:0.2;

the reaction is carried out at room temperature; and

after the reaction is completed, dilute hydrochloric acid is added to adjust the pH to 6-7, the solvent is removed by rotary evaporation. Water is added to the reaction solution, the obtained system is stirred, filtered, and washed with water. The filter cake is collected and dried at 50°C to obtain the compound 6B.

The present application further provides a mixture of formula 8 (cis-trans isomer mixture 8):

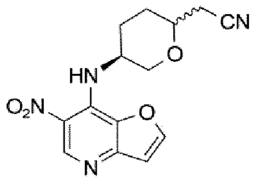

8

,

or salts of the mixture of formula 8, wherein the mixture of formula 8 comprises a compound of formula 3a and a compound of formula 8A:

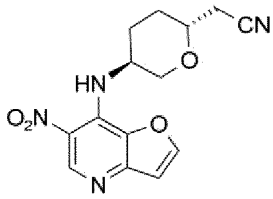

3a

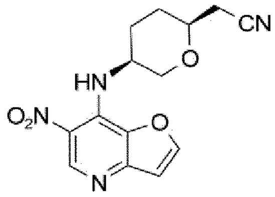

8A

.

The present application further provides a mixture of formula 9 (cis-trans isomer mixture 9):

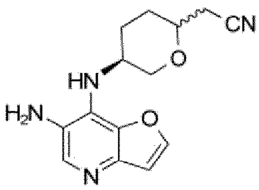

9

,

or salts of the mixture of formula 9, wherein the mixture of formula 9 comprises a compound of formula 4a and a compound of formula 9A:

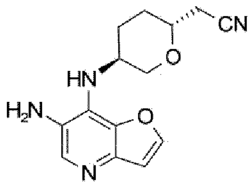

4a

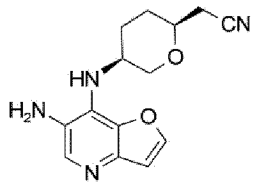

9A

.

The present application further provides a mixture of formula 6Aa (mixture 6Aa):

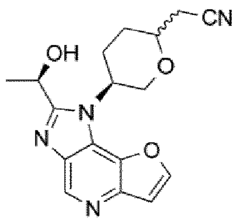

**6Aa**

,

or hydrates of the mixture of formula 6Aa, or salts of the mixture of formula 6Aa, wherein the mixture of formula 6Aa comprises a compound of formula 6A and a compound of formula 6Ab:

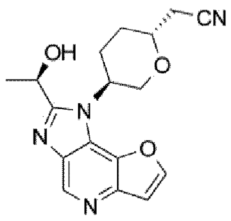

**6A**

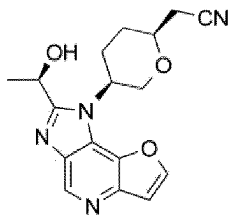

**6Ab**

.

The present application further provides a mixture of formula 6Ba (mixture 6Ba):

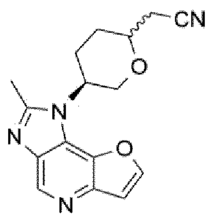

**6Ba**

,

or salts of the mixture of formula 6Ba, wherein the mixture of formula 6Ba comprises a compound of formula 6B and a compound of formula 6Bb:

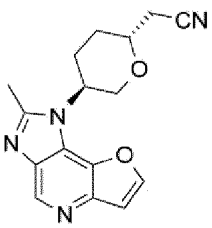

**6B**

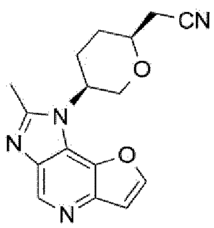

**6Bb**

.

<u>Beneficial effects</u>

The methods for synthesizing the 1H-furo[3,2-b]imidazo[4,5-d]pyridine compounds of the present invention have the advantages of high yield, less impurities and are easy to control, save the cost. The methods are simple, convenient to operate, and are suitable for industrial scale.

## DESCRIPTION OF THE DRAWINGS

FIG. 1 is the $^1$H NMR spectrogram of compound 1 prepared in Example 6 of the present invention.

FIG. 2 is the LCMS spectrogram of compound 1 prepared in Example 6 of the present invention.

FIG. 3 is the $^1$H NMR spectrogram of compound 2a prepared in Example 8 of the present invention.

FIG. 4 is the TsCl-derived HPLC spectrogram of compound 2a prepared in Example 8 of the present invention.

FIG. 5 is the Nα-(2,4-dinitro-5-fluorophenyl)-L-valinamide-derived HPLC spectrogram of compound 2a prepared in Example 8 of the present invention.

FIG. 6 is the $^1$H NMR spectrogram of compound 6A prepared by method A in Example 13 of the present invention.

FIG. 7 is the HPLC spectrogram of compound 6A prepared by method A in Example 13 of the present invention.

FIG. 8 is the HPLC spectrogram of the isomer of compound 6A prepared by method A in Example 13 of the present invention.

FIG. 9 is the $^1$H NMR spectrogram of compound 6B prepared by method A in Example 15 of the present invention.

FIG. 10 is the HPLC spectrogram of compound 6B prepared by method A in Example 15 of the present invention.

FIG. 11 is the $^1$H NMR spectrogram of the tartrate of compound 6B prepared in Example 16 of the present invention.

FIG. 12 is the HPLC spectrogram of the tartrate of the compound 6B prepared in Example 16 of the present invention.

FIG. 13 is the HPLC spectrogram of compound 1 prepared in Example 6 of the present invention.

FIG. 14 is the $^1$H NMR spectrogram of compound 6C prepared in Example 14 of the present invention.

FIG. 15 is the TGA spectrogram of compound 6A prepared in Example 13 of the present invention.

## EMBODIMENT

The sources of main materials in the following examples are shown in the following table:

| Material name | Source |
|---|---|
| Compound 1-1 | Rivocean Jiangsu Pharmaceutical |
| Compound 2-1 | Hangzhou Allsino Chemicals |
| Toluene | Letian Chemistry |
| Sodium carbonate | Hangzhou Longshan Chemical |
| Sodium chloride | Jiangxi JINGHAO Salt Chemical |
| Anhydrous sodium sulfate | Huaian Nanfeng Salt Chemical |
| Triphenylphosphine | Shaoxing Huawei Chemical |
| Dimethylsulfoxide | Hubei Xingfa Chemicals |
| Sodium tert-butoxide | Changzhou GenChem & GenPharm |
| Ethyl formate | Linmu Huasheng Chemical |
| Ethyl acetate | Jinyimeng Group |
| Hydrochloric acid | Lanxi Xuri Chemical |
| Normal heptane | Yueyang Jiaxing Chemical |
| Dichloromethane | Zhejiang Juhua |
| Boron tribromide | Shandong Yanfeng New Material Technology |
| Absolute ethyl alcohol | Jiangsu Huating Biotechnology |

(continued)

| Material name | Source |
|---|---|
| 18% sodium ethoxide/ethanol | Linyi Zhenfeng Chemical |
| Methyl tert-butyl ether | Sinopec |
| Benzoyl chloride | Jiangsu Wanlong Chemistry |
| Sulphuric acid | Zhejiang Jiangtong Fuve Heding Copper |
| 25-28% aqueous ammonia | Hangzhou Allsino Chemicals |
| Acetic acid (glacial acetic acid) | Shanghai Wujing Chemical |
| Acetic anhydride | Ningbo Wanglong Technology |
| 98% nitric acid | Lanxi Xuri Chemical |
| Methyl tert-butyl ether | Sinopec Qilu Petrochemical Company |
| Sodium bicarbonate | Hangzhou Longshan Chemical |
| Diatomite | Yixing Junlian Diatomite |
| Normal heptane | Yueyang Taixin Chemical |
| Tetrahydrofuran | Jiangsu Changchun Chemical |
| Sodium methoxide in methanol solution | Anhui Jinbang Chemical |
| Ammonium chloride | Zhejiang Dayang Biotech |
| Activated carbon | Liyang Jiangyang Activated Carbon Factory |
| DIPEA | Hangzhou Xinde Environmental Protection Technology |
| Acetonitrile | Shanghai SECCO |
| Reduced iron powder | Jiangsu Feixing Powder Metallurgy |
| Acetic acid | Yankuang Lunan Chemicals |
| Sodium citrate | Weifang ENSIGN Industry |
| Potassium phosphate | Jiangsu KOLOD Food Ingerdients |
| Silica gel | Rushan Shangbang New Material |
| Isopropyl alcohol | Zhejiang Xinhua Chemical |
| R-lactic acid | Musashino Chemical |
| Methylcyclohexane | Jiangsu Yangnong Chemical |
| 2-(trans-4-aminocyclohexyl)acetonitrile | Bidepharm |

**Test and inspection methods of compound 1**

Test method 1

Instruments:

Agilent1200 high performance liquid chromatograph or similar liquid chromatograph
Chromatography column: Poroshell 120 EC-C18 (4.6 × 50 mm × 2.7 μm)

Method:

Area normalization method was used for the test.
Chromatography analysis conditions were as follows:
Flow rate: 1.0 ml/min

Column temperature: 30°C
Detector: UV (210 nm)
Post-run: 1 minute

| Mobile phase | A: 100% water + 0.1% TFA; and B: 100% acetonitrile | |
|---|---|---|
| Time (min) | A% | B% |
| 0 | 95 | 5 |
| 5.0 | 5 | 95 |
| 6.67 | 5 | 95 |
| 6.70 | 95 | 5 |
| 8.33 | 95 | 5 |

Test method 2

Testing moisture with Karl Fischer method:

According to the operating procedures of the moisture determination method, about 0.2-0.5g of a test sample of compound 1 was weighted, and the result was accurate to 0.1 mg. The test sample was added into a titration cup of a titrator for determination. The result was recorded. The test sample was determined twice in parallel with the same method, an average value of the two results was taken as the moisture content of the test sample, and one decimal place of the result was kept.

Test method 3

Gas chromatograph, such as Shimadzu GC-2014C or similar gas chromatograph

Gas column: HP-5 30m × 320 μm × 0.25 μm column.

Column temperature: 40°C, kept for 3 minutes; and heated to 260°C at 10°C/min, kept for 2 minutes

Injector temperature: 230°C

Detector temperature: 260°C

Air flow: 400 mL/min

Hydrogen flow: 40 mL/min

Tail blowing nitrogen: 25ml/min

Chromatography column flow (nitrogen): 1 mL/min

Test method 4

Weight loss on drying:

A test sample of evenly mixed compound 1 (if the sample was a relatively large crystal, it should be quickly mashed into particles of about 2mm) was taken and 1-2 gram of the sample was accurately weighed, and placed in a dried flat shape weighing bottle with a plug under the same condition as the sample, the weighing bottle was weighed while it is empty first, and a bottle cup was covered after the sample was added, and then a total weight was weighed. The weighing bottle was shaken parallelly in a gentle way, so that the sample could be evenly spread as much as possible, and the thickness of the sample should not exceed 5 mm. The weighing bottle with the sample was placed in a drying oven under

constant temperature and reduced pressure (the bottle cap was taken out and put in a dryer) and dried at 50°C for 4 hours at -0.09 - -0.1 MPa. The dried sample in the oven was covered with the bottle cap immediately after drying, moved into a dryer, cooled to room temperature, and then weighed.

The calculation formula of the weight loss on drying

$$\text{Weight loss on drying} = (W1\text{-}W2)/W1*100\%.$$

W1 was the sample weight before drying, and W2 was the sample weight after drying.

Method for detecting finished product of compound 1

1. Appearance

This product was put under natural light for visual inspection.

2. Detection

A nuclear magnetic resonance method was employed

Detection was conducted by an external unit according to USP<761> by using $CDCl_3$ as the solvent.

3. Purity and related substances

Instrument and equipment

| Name | Specifications and model |
|---|---|
| High-Performance liquid chromatography | DAD or VWD system, Agilent 1260 or equivalent |
| Analytical balance | METTLER TOLEDO XPR205 or equivalent instrument, d = 0.01 mg or above |
| Chromatography column | Agilent Zorbax SB C18 4.6 mm × 250 mm, 5 μm or similar columns |
| Ultrapure water system | Milli-Q IQ7000 or equivalent instrument |

Reagent

| Name | Grade/purity |
|---|---|
| Acetonitrile (ACN) | HPLC grade or equivalent |
| Pure water | Milli-Q grade or equivalent |
| Trifluoroacetic acid (TFA) | HPLC grade or equivalent |

Sample information

| | |
|---|---|
| Sample | Test sample of compound 1 |

Chromatographic condition

| | | | |
|---|---|---|---|
| Instrument | Agilent 1260 HPLC System | | |
| Chromatography column | Agilent Zorbax SB C18 4.6 mm × 250 mm, 5 μm or similar columns | | |
| Mobile phase A | 0.1% TFA aqueous solution | | |
| Mobile phase B | Acetonitrile | | |
| Column temperature | 25°C | | |
| Flow rate | 1.0 ml/min | | |
| Detector | DAD or VWD | | |
| Wavelength | 300 nm | | |
| Injection volume | 5 μL | | |
| Data acquisition time | 37 min | | |
| Gradient | Time (min) | A (%) | B (%) |
| | 0 | 90 | 10 |
| | 20 | 10 | 90 |
| | 30 | 10 | 90 |
| | 30.1 | 90 | 10 |
| | 37 | 90 | 10 |

**Detection method of compound 2a**

Determination of compound 2a by GC chromatographic detection

| | |
|---|---|
| Instrument | Shimadzu GC-2014C |
| Chromatography column | Agilent DB1701 30 m × 0.32 mm × 0.25 μm |
| Injection temperature | 260°C |
| Detector temperature | 280°C |
| Detector | FID |
| Injection volume | 5.0 μL |
| Temperature program | Initial temperature:50°C for 1 min<br>Heated to 280°C at a rate of 10°C/min<br>Maintain at 280°C for 3 mins |
| Carrier gas flow rate (in column) | 1.87 mL/min |
| Split ratio | 30:1 |
| Gas Flow | H2:40 mL/ min; Air:400 mL/ min |
| Exhaust gas purging | 30 mL/ min |

Determination method of HPLC purity and d.e value of finished product of compound 2a

| | | | |
|---|---|---|---|
| Operating procedure | A mixture of 100 mg of compound 2a, 1 mL of acetonitrile, 1 mL of water, 120 mg of sodium carbonate and 129.5 mg of p-toluenesulfonyl chloride was stirred at room temperature for 1-2 hours, then sampled and detected by HPLC.<br>A mixture of 100 mg of compound 2a-diastereomer standard sample, 1 mL of acetonitrile, 1 mL of water, 120 mg of sodium carbonate and 129.5 mg of p-toluenesulfonyl chloride was stirred at room temperature for 1-2 hours, then sampled and detected by HPLC (control). | | |
| Instrument | Agilent1260 HPLC System or Similar configuration | | |
| Column | Agilent Eclipse EC-C18 (4.6mm × 150mm, 2.7 μm) | | |
| Column temperature | 30°C | | |
| Detector wavelength | 220 nm | | |
| Mobile phase velocity | 1.0 ml/min | | |
| Mobile phase A | 0.1% TFA aqueous solution | | |
| Mobile phase B | Acetonitrile | | |
| Diluent | Acetonitrile | | |
| Running time | 25.00 min | | |
| Gradient elution procedure | Time (min) | A% | B% |
| | 0 | 95.0 | 5.0 |
| | 15.0 | 5.0 | 95.0 |
| | 20.0 | 5.0 | 95.0 |
| | 24.0 | 95.0 | 5.0 |
| | 25.0 | 95.0 | 5.0 |
| Compound ID | Retention time (min) | | |
| Compound 2a | 9.9 | | |
| Diastereomer of compound 2a | 10.1 | | |

Determination method of e.e value of finished product of compound 2a

| | |
|---|---|
| Operating procedure | 100 mg of compound 2a, 30 ml of tetrahydrofuran and 1.61 g of N,N-diisopropylethylamine were added, the mixture was stirred evenly and then 1.87 g of Z8032 derivative reagent was added to the mixture, and the obtained mixture was stirred at 20-30°C for 1-2 hours, then samples were detected by HPLC.<br>100 mg of compound 2a-enantiomer standard sample, 30 ml of tetrahydrofuran, 1.61 g of N,N-diisopropylethylamine were added, the mixture was stirred evenly and then 1.87 g of Z8032 derivative reagent was added to the mixture, and the obtained mixture was stirred at 20-30°C for 1-2 hours, then samples were detected by HPLC (control) |
| Instrument | Agilent1260 HPLC System or Similar configuration |
| Column | Agilent Poroshell 120 EC-C18 (4.6mm × 100mm, 2.7 μm) |

(continued)

| | | | |
|---|---|---|---|
| Column temperature | 35°C | | |
| Detector wavelength | 214 nm | | |
| Mobile phase velocity | 1.2 ml/min | | |
| Mobile phase A | 0.1% TFA aqueous solution | | |
| Mobile phase B | Acetonitrile | | |
| Diluent | Acetonitrile | | |
| Running time | 25.00 min | | |
| Gradient elution procedure | Time (min) | A% | B% |
| | 0.0 | 85.0 | 15.0 |
| | 15.0 | 40.0 | 60.0 |
| | 16.0 | 5.0 | 95.0 |
| | 21.0 | 5.0 | 95.0 |
| | 21.1 | 85.0 | 15.0 |
| | 25.0 | 85.0 | 15.0 |
| Compound ID | Retention time (min) | | |
| Compound 2a | 11.7 | | |
| Enantiomer of compound 2a | 12.0 | | |

**Detection methods of compounds 3a, 4a and 6A**

Detection method 1

| **Test purpose** | **Intermediate control of compound 3a and compound 4a and purity analysis of product** |
|---|---|
| **Instrument model** | Agilent 1200 |
| **Column model** | Agilent Eclipse XDB-$C_{18}$ (4.6 mm × 150 mm × 5.0 μm) |
| **Column temperature** | 35°C |
| **Detector wavelength** | 220 nm |
| **Mobile phase velocity** | 1.0 mL/min |

(continued)

| Test purpose | **Intermediate control of compound 3a and compound 4a and purity analysis of product** | |
|---|---|---|
| **Mobile phase solvent A** | 0.05% TFA aqueous solution | |
| **Mobile phase solvent B** | 100% acetonitrile | |
| **Running time** | 30 min | |
| **Post-running time** | 1 min | |
| **Gradient elution procedure** | Time (min) | B% |
| | 0 | 5 |
| | 15 | 25 |
| | 22 | 95 |
| | 25 | 95 |
| | 25.1 | 5 |
| | 30 | 5 |
| | | |
| **Compound number** | **Retention time (min)** | |
| **Compound 1** | 19.43 | |
| **Compound 3a** | 15.56 | |
| **Compound 4a** | 9.52 | |

Detection method 2

| | |
|---|---|
| **Detection purpose** | Intermediate control of compound 6A and purity analysis of product |
| **Instrument model** | Agilent 1200 |
| **Column model** | Poroshell 120 PheHex (4.6mm × 250 mm × 4.0 μm) |
| **Column temperature** | 40°C |
| **Detector wavelength** | 220 nm |
| **Mobile phase velocity** | 1.0 mL/min |
| **Mobile phase solvent A** | 20 mmol ammonium acetate aqueous solution |
| **Mobile phase solvent B** | 100% methanol |
| **Running time** | 45 min |
| **Post-running time** | 1 min |

(continued)

| | | |
|---|---|---|
| Gradient elution procedure | Time (min) | B% |
| | 0 | 30 |
| | 14 | 53.5 |
| | 30 | 75 |
| | 35 | 90 |
| | 40 | 90 |
| | 40.1 | 30 |
| | 45 | 30 |
| | | |
| **Compound ID** | **Retention time (min)** | |
| **Compound 4a** | 10.47 | |
| **Compound 6A** | 14.6 | |

Detection method 3

| | | |
|---|---|---|
| **Detection purpose** | Purity Analysis of isomer of compound 6A | |
| **Instrument model** | Agilent 1200 | |
| **Column model** | CHIRALPAK AD-H (4.6mm × 150 mm × 5.0 μm) | |
| **Column temperature** | 40°C | |
| **Detector wavelength** | 244 nm | |
| **Mobile phase velocity** | 1.0 mL/min | |
| **Mobile phase solvent A** | Normal hexane | |
| **Mobile phase solvent B** | Ethanol | |
| **Running time** | 45 min | |
| **Post-running time** | 1 min | |
| **Gradient elution procedure** | Time (min) | B% |
| | 0 | 30 |
| | 45 | 30 |
| | | |
| **Compound ID** | **Retention time (min)** | |
| **Compound 6A** | 10.99 | |
| **Isomer of compound 6A** | 21.33 | |

Detection method 4

| | |
|---|---|
| **Analysis purpose** | Determination of residual solvent in finished baking material |
| **Instrument model** | Agilent 7890A gas chromatograph |

(continued)

| | |
|---|---|
| **Chromatography column information** | Agilent DB-624 (30 m × 0.53 mm ID × 3.0 μm) |
| **Injection temperature** | 200°C |
| **Detector temperature** | 250°C |
| **Detector** | Flame ionization detector FID |
| **Column oven programmed temperature** | The initial temperature was 45°C, and the temperature was kept for 5 minutes, then the system was heated to 120°C at a rate of 10°C per minute, and the temperature was kept for 5 minutes, and then the system was heated to 220°C at a rate of 35°C per minute, and the temperature was kept for 3 minutes. |
| **Flow rate in the column** | 2 mL/ min |
| **Gas flow rate** | Air:400 ml/min<br>Hydrogen:40 ml/min<br>Auxiliary gas flow rate:25.0 ml/min. |
| **Blank solvent** | DMF (N,N-dimethylformamide) |
| **Diluent** | DMF(N,N-dimethylformamide) |
| **Injection volume:** | 2 μl |
| **Split ratio:** | 20:1 |
| **Carrier gas:** | Helium or nitrogen |
| **Data acquisition time:** | 30 min |
| **Analytical balance** | Accurate to 0.01 mg |
| **Grade of volumetric flask** | Grade A |

**Detection method for compound 6B and the tartrate of compound 6B**

Chromatograph: Agilent 1260 high performance liquid chromatograph.

Column model: Waters XBridge C18 250 mm × 4.6 mm × 5 μm.

Mobile phase A: 20 mM dipotassium hydrogen phosphate aqueous solution

Mobile phase B: methanol

Gradient:

| Time (min) | Mobile phase A(%, V/V) | Mobile phase B(%, V/V) |
|---|---|---|
| 0 | 80 | 20 |
| 20 | 50 | 50 |

(continued)

| Time (min) | Mobile phase A(%, V/V) | Mobile phase B(%, V/V) |
|---|---|---|
| 30 | 30 | 70 |
| 40 | 30 | 70 |
| 40.01 | 80 | 20 |
| 50 | 80 | 20 |

Column temperature: 40°C

Sample room temperature: room temperature

Flow rate: 1 mL/min

Injection volume: 5 μL

Running time: 50 min

Test sample concentration: about 1 mg/mL

Detection wavelength: UV 230 nm.

The present invention is further illustrated hereinafter with reference to the following examples, but the examples do not constitute a limitation or restriction on the scope of the present invention.

**Example 1: Preparation of compound 1-2**

2-chloromethyl-3,4-dimethoxypyridinium hydrochloride (250 kg, 1.0 eq) was added to reaction vessel 1 under the protection of nitrogen, and toluene (1050 kg) and drinking water (150 kg) were added. The reaction solution was stirred and cooled to 20-25°C. Sodium carbonate (68.8 kg, 0.58 eq) was added in batches, the temperature in the vessel was controlled to be less than or equal to 30°C, then the reaction solution was stirred for 0.5 hour. The system was allowed to settle for 20 minutes and layered. The upper organic phase was reserved, and the lower aqueous phase was transferred to reaction vessel 2 and extracted with toluene (435 kg), the organic phase was combined in the reaction vessel 1. The aqueous phase was extracted with toluene (225 kg) again, the lower aqueous phase was discarded, and the organic phase was combined in the reaction vessel 1. The combined organic phase was washed with brine (204 kg) and dried with anhydrous sodium sulfate (150 kg). The obtained filtrate after filtering was transferred to reaction vessel 3, and the obtained solid was rinsed with toluene (50 kg). Triphenylphosphine (350 kg, 1.2 eq) was added to the reaction vessel 3, the system was stirred, the internal temperature was heated to 108-115°C, the reaction solution was refluxed with a Dean-Stark apparatus for 18 hours. After the reaction was completed, the materials in the reaction vessel 3 were cooled to 20-30°C. The materials were centrifuged, and the obtained solids were rinsed with toluene (100 kg). The wet product was dried with double cones, the temperature of the hot water in the jacket was 50-60°C, a vacuum degree was below -0.090 Mpa, until water was less than or equal to 0.2%. The product was collected, packed with two-layer PE bags and put into a fibreboard drum (output: 489.8 kg, yield: 97.4%, and HPLC purity: 99.8%).

**Example 2: Preparation of compound 1-3**

Compound 1-2 (489.8 kg, 1.0 eq), and dimethylsulphoxide (1614.2 kg, 3V) were added in reaction vessel 1, stirred, and then cooled to 20-25°C. Sodium tert-butoxide (114.9 kg, 1.1 eq) was added in batches, the obtained system was heated to 25-32°C after the addition. The reaction solution was stirred for 1.5 hours with the temperature kept at 25-32°C. Ethyl formate (397.8 kg, 5.0 eq) was dropwise added to the reaction vessel 1, the reaction vessel 1 was heated to 40-50°C, the reaction solution was stirred for 8 hours with the temperature kept at 40-50°C. After the reaction was completed, the reaction vessel 1 was cooled to a temperature less than or equal to 25°C. The reaction mixture was extracted with ethyl acetate (1320.7 kg, 3V) and drinking water (1467.4 kg, 3V), the obtained organic layer was separated, the obtained aqueous phase was extracted with ethyl acetate twice. The organic phases were combined, and washed with water (978.3 kg). Water (1467.4 kg) was added to the organic phase and hydrochloric acid (108.7 kg) was dropwise added to adjust the pH to 5-6. The organic phase was separated and washed with water (250.0 kg), the aqueous phases were

combined and extracted with ethyl acetate (1320.2 kg) twice. To the aqueous phase was added ethyl acetate (1320.7 kg, 3V) and sodium carbonate (54.0 kg) in batches to adjust the pH to be greater than or equal to 8. The organic phase was separated, and the obtained aqueous phase was extracted with ethyl acetate (880.4 kg, 2V). The combined organic phases were washed with brine (341.3 kg), dried with anhydrous sodium sulfate (217.4 kg), filtered under vacuum, and rinsed with ethyl acetate (81.5 kg). The filtrate was collected and the solvent was removed. The residue was triturated with n-heptane (337.0 kg) and centrifuged. The obtained solid was rinsed with n-neptane (76.0 kg), and dried to obtain the compound 1-3 (output: 202.0 kg, yield: 88.8%, and HPLC purity: 97.3%).

**Example 3: Preparation of compound 1-4**

Compound 1-3 (200.0 kg, 1.0 eq) and dichloromethane (1431.0 kg, 6 V) were added in reaction vessel 1 under the protection of nitrogen, keeping the temperature of the materials in the reaction vessel at 15-20°C. Boron tribromide (600.0 kg, 2.5 eq) was added, keeping the temperature of the materials in the reaction vessel at 20-30°C. After the addition, the reaction solution was stirred for 1 hour with the temperature kept at 20-30°C. At 15-30°C, and under the protection of nitrogen, the mixed solution in the reaction vessel 1 was slowly added dropwise into reaction vessel 2 containing ethyl alcohol (1422.0 kg, 10 V). The reaction system was stirred for 2 hours with the temperature kept at 30-35°C. The solvent was removed under reduced pressure to 1600 L. To the resulting solution was added ethyl alcohol (570.0 kg 4 V) twice and concentrated to volume of 1,600 L for each addition. The temperature was controlled at 15-30°C, and 18% sodium ethoxide in ethanol solution (1367.0 kg) was added to adjust the pH to 5-6. The solid was separated and rinsed with anhydrous ethanol (143.0 kg, 1 V). To the mother liquid was added 18% sodium ethoxide in ethanol (1378.4 kg) to adjust the pH to 8-9. The solvent was removed under reduced pressure, and methyl tert-butyl ether (540.0 kg, 4 V) was added. The solid was separated, rinsed with methyl tert-butyl ether (135.0 kg, 1 V), and dried to obtain the compound 1-4 (output: 170 kg, yield: 78.3%, and HPLC purity: 97.9%).

**Example 4: Preparation of compound 1-5**

Benzoyl chloride (214.6 kg, 2.0 eq) was slowly dropwise added to a dichloromethane solution (3271.0 kg, 25.5 V) containing compound 1-4 (170.0 kg, 1.0 eq) at 0-10°C. The reaction solution was stirred at 0-10°C for 1 hour. At 15-25°C, the reaction solution was slowly added dropwise into a reaction vessel containing sulfuric acid (1518.0 kg, 4.85 V), and the resulting mixture was stirred for 1 hour. The reaction solution was quenched with water (850.0 kg, 5 V) at 0-10°C. The solid was separated and rinsed with water (85.0 kg, 0.5 V), and the mother solution was cooled to 0-10°C and dropwise added with aqueous ammonia (1841.3 kg) to adjust pH to 7-8. The mixture was stirred at 53-57°C for 0.5 hour and then cooled to 20-25°C. The solid were separated and rinsed with water (170.0 kg, 1 V). The thus obtained solids were dried to obtain the compound 1-5 (output: 92.7 kg, yield: 91.7%, and HPLC purity: 98.3%).

**Example 5: Preparation of compound 1-6**

Nitric acid (52.5 kg) was dropwise added into reaction vessel 1 containing acetic acid (141.0 kg) and acetic anhydride (89.25 kg) at 15-30°C and stirred at 20-30°C for 0.5 hour. The mixed solution was slowly added dropwise into an acetic acid solution (450.0 kg, 10W) containing compound 1-5 (45.0 kg, 1.0 eq) at the temperature of 113-116°C under the protection of nitrogen for more than 2 hours, keeping the temperature at 112-117°C. The mixed solution was stirred for 1 hour with the temperature kept at 112-117°C. After the temperature was cooled to 20-30°C, methyl tert-butyl ether (337.5 kg, 10 V) was added and the mixture was stirred for 0.5 hour. The solid was separated and rinsed with methyl tert-butyl ether (100.8 kg 3 V). The thus obtained solids were dried to obtain the compound 1-6 (output: 44.4 kg, yield: 74.0%, and HPLC purity: 94.7%).

**Example 6: Preparation of compound 1**

Phosphorus oxychloride (72.8 kg, 1.2 eq) was dropwise added into an N,N- dimethylformamide solution (214.2 kg, 3.0 W) containing compound 1-6 (71.8 kg) at 15-25°C under the protection of nitrogen. The reaction mixture was stirred at 38-43°C for 1 hour and then cooled to 15-20°C. The reaction solution was dropwise added into water (714.0 kg, 10 W) for quenching, and the reaction solution was stirred for 1 hour with the temperature kept at 20-30°C. The solid was separated, rinsed with water (214.2 kg, 3 W), and then stirred in dichloromethane (1224.0 kg, 13 V) for 15 minutes. 5% sodium bicarbonate aqueous solution (357.0 kg) and celite (35.7 kg) were added, and the mixture was stirred for 0.5 hour. The solid was separated and rinsed with dichloromethane (195.3 kg, 2 V). The filtrate was collected. The dichloromethane layer was separated, and the water layer was extracted with dichloromethane (474.3 kg, 5 V). Dichloromethane layers were combined, washed with brine (485.5 kg), dried with anhydrous sodium sulfate (107.0 kg, 1.5 W) for 1 hour, filtered, and rinsed with dichloromethane (188.7 kg, 2V). The filtrate was collected and activated carbon (7.0 kg, 0.1 W) was added. The

resulting mixture was stirred for 1 hour at 20-30°C, filtered and rinsed with dichloromethane (93.8 kg, 1 V). The solvents were removed, and n-heptane (242.8 kg, 5 V) was added for trituration. The separated solid was rinsed with n-heptane (47.9 kg, 1 V) and dried to obtain the compound 1 (output: 62.2 kg, yield: 78.5%, and HPLC purity: 99.96%). The $^1$H NMR spectrogram was shown in FIG. 1, the LCMS spectrogram was shown in FIG. 2, and the HPLC spectrogram was shown in FIG. 13.

**Example 7: Preparation of compound 2-2**

A methanol solution of sodium methoxide (19.18 kg, 0.25 eq) and tetrahydrofuran (91.1 kg, 1.0 V) were added to a tetrahydrofuran solution (911.4 kg, 10 V) containing compound 2-1 (102.4 kg, 1.0 eq) at 0-5°C.The reaction was stirred at 20-25°C for 4-8 hours. The reaction was monitored by GC and HPLC until the starting material was less than or equal to 1.0%. The temperature was reduced to 15-20°C, and an aqueous solution (13.3 kg, 0.13 V) of ammonium chloride (5.69 kg, 0.25 eq) was added to the reaction to adjust the pH to 7-8, keeping the temperature at 15-25°C. Methyl tert-butyl ether (166.7 kg, 2.2 V) and 20% sodium chloride solution (61.4 kg sodium chloride, 0.6 W and 245.8 kg drinking water, 2.4 W) were added to the mixed solution respectively. The organic phase was separated, and the aqueous phase was extracted with methyl tert-butyl ether (75.8 kg, 1 V), and the organic phases were combined. The organic phases were washed with 30% sodium chloride aqueous solution (43.0 kg,0.42 W sodium chloride solid, 100.4 kg, 0.98 W drinking water), and then separated. The aqueous phase was extracted with methyl tert-butyl ether (75.8kg, 1V). The organic phases were combined, and anhydrous sodium sulfate (51.2 kg, 0.5 W) and activated carbon (10.24 kg, 0.1 W) were added. The mixture was filtered and rinsed with methyl tert-butyl ether (41.0 kg, 0.4W). The filtrate was collected, concentrated, and added methyl tert-butyl ether (227.3 kg, 3.0 V). The mixture was triturated at 50-55°C for 2 hours, and slowly cooled to 0-10°C. The thus obtained solid was filtered, collected and dried to obtain the compound 2-2 (output: 43.2 kg, and yield: 42.2%).

Quality standards of compound 2-2: appearance: off-white solid; purity (GC): 96.0%; maximum unknown single impurity: 1.6%; and moisture: 0.0%.

**Example 8: Preparation of compound 2a hydrochloride**

Compound 2-2 (43.21 kg, 1.0 eq) and dichloromethane (192 kg, 6 V) were added to reaction vessel R01and the obtained system was cooled to 0-5°C; then 35% hydrogen chloride in ethanol solution (hydrogen chloride:19.3 kg, absolute ethyl alcohol: 35.9 kg) was dropwise added. After the dropwise addition, the system was heated to 20-25°C, and the temperature was kept for 4-8 hours. Upon detection by TLC and GC, the system was cooled to 0-5°C after the content of the raw material compound 2-2 was less than or equal to 1.0%. The system was centrifuged to obtain a wet product of the hydrochloride of the compound 2a, and then the wet product was rinsed with dichloromethane (32.0 kg, 1 V), and centrifuged to dryness; double-cone jacket was at 55-65°C and the obtained material was dried under vacuum with a vacuum degree of less than or equal to - 0.095 MPa for 12 hours to obtain the compound 2a hydrochloride (output: 28.96 kg, and yield: 91.2%). The $^1$H NMR spectrogram was shown in FIG. 3, the TsCl-derived HPLC spectrogram was shown in FIG. 4, and the Nα-(2,4-dinitro-5-fluorophenyl)-L-valinamide-derived HPLC spectrogram was shown in FIG. 5.

Quality standards of the compound 2a hydrochloride: appearance: white solid; purity (TsCl-derived HPLC): 99.8%; d.e value (TsCl-derived HPLC): 99.8%; e.e value (Nα-(2,4-dinitro-5-fluorophenyl)-L-valinamide-derived HPLC): 99.9%; maximum single impurity: 0.2%; and moisture:0.1%.

**Example 9: Preparation of compound 3a**

Method A

Anhydrous ethanol (110.6 kg, 5 V), acetonitrile (109.2 kg, 5 V), compound 1 (27.97 kg, 1.0 eq) and the compound 2a hydrochloride (25.07 kg, 1.0 eq) were pumped into a 1000L reaction vessel (R01), and then N,N-diisopropylethylamine (40.0 kg, 2.2 eq) was pumped into the mixture under the protection of nitrogen. The reaction solution was reacted at 50-60°C for 16 hours, and samples were tested by HPLC until compound 1 was not higher than 1.0%. The reaction solution was cooled to 20-30°C, and water (560 kg, 20 V) was dropwise added at 20-30°C. After the dropwise addition, the reaction solution was continuously stirred for 1.0 hour with the temperature kept at 20-30°C. The materials were centrifuged after stirred for 2 hours at 0-5°C, and the filter cake was rinsed once with a mixed solvent of acetonitrile and water (pre-cooled at 0-10°C, 25 kg of acetonitrile + 56 kg of water). The filter cake was dried in a vacuum oven at 45-55°C to obtain the compound 3a (output: 37.51 kg, moisture: 0.03%, and yield: 88.1%).

Method B

To a flask was added with ethanol (1.9 L), compound 1 (200 g, 1.0 eq), compound 2a hydrochloride (179.7 g, 1.01 eq) and sodium bicarbonate (188 g, 2.23 eq) under nitrogen atmosphere. The reaction solution was reacted at 70-80°C for 16 hours. The reaction solution was cooled to 20-30°C, and water (2.9 L) was slowly added. The resulting mixture was stirred for 2 hours, and cooled to 5-15°C. The mixture was stirred for 4 hours with the temperature kept at 5-15°C, and filtered. The filter cake was eluted with water and ethanol (600 mL, water/ethanol = 1:2), and dried to obtain the compound 3a as a yellow solid (output: 286.9 g, and yield: 94%).

Method C

The compound 3a was prepared by the same preparation method as that of the method B, except that the added base was different from that of the method B, wherein the added base was sodium carbonate (2.2 eq), and the compound 3a detected by HPLC was 72%.

Method D

The compound 3a was prepared by the same preparation method as that of the method B, except that the added base was different from that of the method B, wherein the added base was potassium bicarbonate (2.2 eq), and the compound 3a detected by HPLC was 83%.

Method E

Anhydrous ethanol (1 mL), acetonitrile (1 mL), compound 1 (200 mg, 1.0 eq), compound 2a hydrochloride (179 mg,1.0 eq), 1,8-diazabicyclo[5.4.0]undec-7-ene (339 mg, 2.2 eq) were added to a 40 mL flask, and reacted for 16 hours at 55°C. Then, the reaction solution was cooled to room temperature, concentrated and separated by column chromatography to obtain the compound 3a (output: 198 mg, and yield: 65.1%).

**Example 10: Preparation of compound 3b**

Under nitrogen atmosphere, ethanol (27 mL), compound 1 (2.84 g, 1.0 eq), compound 2b (2-(trans-4-aminocyclohexyl)acetonitrile, 2 g, 1.01 eq) and sodium bicarbonate (1.44 g, 1.2 eq) were added to a flask, and reacted at 75°C for 16 hours. The reaction solution was cooled to 20-30°C, water (30 mL) was added slowly and the obtained system was stirred for 2 hours, and filtered. The filter cake was eluted with water and ethanol (6 mL, water/ethanol = 1:2), and dried to obtain the compound 3b as a yellow solid (output: 3.694 g, and yield: 86%).

**Example 11: Preparation of compound 4a/compound 4a hydrochloride**

Method A

Under the protection of nitrogen, acetonitrile (264 kg, 9 V), acetic acid (90 kg, 12 eq) and compound 3a (37.43 kg, 1.0 eq) were added into a 1000L glass-lined reactor. Iron powder (41.17 kg, 6.0 eq) was added in batches at 60-70°C and stirred for 1 hour with the temperature kept at 60-70°C. Iron powder (14 kg, 2.02 eq) and acetic acid (10 kg, 1.34 eq) were added again until the reaction was complete. The reaction solution was cooled to 30-40°C, filtered and eluted with hot acetonitrile (50-60°C, 176 kg, 6 V). 30% sodium citrate aqueous solution (sodium citrate 164.56 kg, 5.15 eq, water 380 kg) was added to the filtrate at 10-25°C until the pH was 6, and then 30% potassium phosphate aqueous solution (potassium phosphate 224.46 kg, 8.5 eq, and water 522 kg) was added until the pH was 8. After vacuum distillation, ethyl acetate (167.8 kg, 5 V) and water (112 kg, 3 V) were added. The organic phase was separated, and the aqueous phase was extracted with ethyl acetate (101 kg, 3 V). The organic phases were combined, washed with 20% sodium citrate aqueous solution (water, 60 kg, and sodium citrate, 15 kg, 0.47 eq) and 15% sodium chloride aqueous solution (water, 61.3 kg, and sodium chloride, 10.8 kg) subsequently. Anhydrous sodium sulfate (41.5 kg) and silica gel (5.23 kg) were added and the resulting mixture was stirred for 30 minutes. The mixture was filtered and rinsed with ethyl acetate (67 kg). After distillation under reduced pressure, 30 kg of isopropanol was added into the system, and evaporated to dryness under reduced pressure, then another 30 kg of isopropanol was added and evaporated to dryness under reduced pressure. 101.15 kg of isopropanol was added, and the thus obtained system was stirred at 80°C for dissolving and obtaining a clear solution. After dissolving to clear, the reaction solution was slowly cooled to 0-10°C and stirred for 2 hours. The thus obtained solid were separated, and the filter cake was washed with 18.72 kg of isopropanol and dried to obtain the compound 4a (21.15kg, yield: 55%). The content of the isopropanol was: 12.65%.

Method B

To a reaction flask was added compound 3a (210 g, 1.0 eq), water (840 mL) and methanol (210 mL), and the temperature was increased to 65-75°C with stirring. Hypoboric acid (218 g, 3.5 eq, added in 7 times) was then added in batches at 65-75°C and the reaction was carried out overnight. Samples were taken for detection (additional hypoboric acid was added until the reaction was completed). After the reaction was completed, the reaction solution was cooled to 35-45°C, part of the solvent was concentrated under reduced pressure, the temperature was decreased to 0-10°C. The thus obtained system was stirred for 1-2 hours, and filtered. The filter cake was eluted with 1000 mL of dichloromethane, the filtrate was collected, and sodium bicarbonate (105 g) was added in batches at 20-30°C and the system was stirred with the temperature kept at 20-30°C for 1 hour, and the system was allowed to settle for 0.5 hour. The obtained organic phase was separated, and the obtained aqueous phase was extracted with dichloromethane (660 mL × 3). The thus obtained organic phases were combined, dried with anhydrous sodium sulfate (210 g). Hydrochloric acid in ethanol solution (230 mL, 30%) was dropwise added at 15-25°C and the obtained system was stirred for 8 hours with the temperature kept at 15-25°C. Ethyl acetate (1050 mL) was dropwise added at 15-25°C and the obtained system was stirred for 1 hour with the temperature kept at 15-25°C, then filtered. The filter cake was eluted with ethyl acetate (420 mL) and dried in a vacuum drying oven at 30-40°C to obtain the compound 4a hydrochloride, i.e., 2-((2R,5S)-5-(6-aminofuro[3,2-b]pyridine-7-ylamino)tetrahydro-2H-pyran-2-yl)acetonitrile hydrochloride (output: 232.8 g, and yield: 85.3%).

Method C

At room temperature, compound 3a (1.00 g, 3.31 mmol), 4,4'-bipyridine (2.58 mg, 0.0165 mmol) and solvent DMF (20 mL) were added into a 50 mL multi-necked flask. The mixture was purged with nitrogen for three times with stirring, and $B_2(OH)_4$ (0.89 g, 9.92 mmol) was added. The mixture was reacted at 15-25°C for 5 hours. The reaction solution was detected with HPLC. After the compound 3a was fully consumed, the reaction was stopped, and 200 mL of water was added. The obtained system was extracted with ethyl acetate five times (50 mL each time), washed with 100 mL of brine, dried with anhydrous sodium sulfate, and concentrated to obtain a crude product. The crude product was further separated by column chromatography with dichloromethane and methanol (30:1 (v/v)) to obtain the compound 4a, with a yield of 44.4%.

Method D

Ethyl acetate (2 mL), compound 3a (198 mg, 1.0 eq) and stannous chloride (373 mg, 3.0 eq) were added into a reaction flask, and reacted at 85°C for 3 hours, then cooled to room temperature. Sodium bicarbonate was added with an ice bath, and the obtained system was stirred and filtered. The filter cake was rinsed with 2 mL of ethyl acetate. The filtrate was collected, and washed with aqueous solution of saturated sodium bicarbonate for three times. The organic phases were combined and then washed with brine for three times. The thus obtained organic phases were combined, dried with anhydrous sodium sulfate, filtered, and concentrated under reduced pressure to obtain the compound 4a (output:123 mg, and yield:69%).

**Example 12: Preparation of compound 4b**

Compound 3b (3.69 g, 1.0 eq), water (14.8 mL) and methanol (3.7 mL) were added into a reaction flask, and the temperature was increased to 65-75°C with stirring. Hypoboric acid (4.51 g, 4.1 eq, added in 4 times) was added in batches, and the mixture was reacted at 65-75°C overnight. After the reaction was completed, the reaction solution was cooled to 20-30°C. Sodium bicarbonate (1.85 g) was added in batches, and the mixture was stirred for 0.5 hour at 20-30°C. The organic phase was separated, and the aqueous phase was extracted with dichloromethane (10 mL × 3), and then the thus obtained organic phases were combined, washed with water (10 mL × 3) and brine (10 mL × 3), dried with anhydrous sodium sulfate (3.7 g), filtered, and concentrated to obtain the compound 4b (output 3.02 g, and yield:90.9%).

**Example 13: Preparation of compound 6A**

Method A

R-lactic acid (30.5 kg, 5.0 eq, isomer content: 0.25%), compound 4a (21.14 kg, 1.0 eq) and methylcyclohexane (37.0 kg) were added into 300L reaction vessel R01, and the obtained system was stirred and heated to reflux at 87.2-101°C for 5 hours with a Dean-Stark apparatus, and then samples were taken for In-Process Control. After the reaction was completed, steam heating was stopped and methanol (43.8 kg, 3 V) was slowly dropwise added into the reaction vessel R01, and the system was cooled to 20-30°C. The reaction solution was allowed to settle for layering. The

lower layer was separated, and methanol (29.6 kg, 2 V) was added. Sodium hydroxide solution (25.3 kg of water, and 10.9 kg of sodium hydroxide) was dropwise added at -10-0°C and the materials were reacted with the temperature kept at -10-0°C until HPLC detection qualified. Hydrochloric acid (4.81 kg of concentrated hydrochloric acid + 45.03 kg of water) was dropwise added to adjust the pH to 7. After the solvent was removed under vacuum, water (277.5 kg, 15 V) was added. The resulting mixture was stirred for 1 hour, and then centrifuged. The filter cake was washed with water (55.5 kg, 3V). The obtained wet product was put into 185 kg of water. The mixture was stirred for 2 hours at about 30°C, centrifuged, washed with 30 kg of water, and dried to obtain a crude product of the compound 6A. HPLC purity of compound 6A: 97.24%; HPLC content of the isomer of compound 6A: 1.95%; water: 5.2%.

Refining compound 6A:

A mixture of 20.49 kg of the crude product of the compound 6A, 116 kg of isopropanol and 50 kg of water was stirred and refluxed for dissolving to clear. The system was cooled to -5 - 5°C, stirred for 3 hours with the temperature kept at -5 - 5°C, and centrifuged. The filter cake was rinsed once with a mixed solvent (isopropanol (12 kg) and water (5 kg), pre-cooled to 0-10°C) to obtain a primary recrystallized compound 6A. HPLC purity of compound 6A: 99.03%; and HPLC content of the isomer of compound 6A: 1.03%. Recrystallization (107.16 kg of isopropanol, and 45.36 kg of water) was carried out again to obtain a secondary recrystallized compound 6A. HPLC purity of compound 6A: 99.66%; HPLC content of the isomer of compound 6A: 0.43%. 87.7 kg of methanol and 1.2 kg of activated carbon were added to 17.1 kg of the wet product of the compound 6A, and the obtained system was refluxed with stirring for 1 hour. The mixture was filtered with pressure (through polishing filter) and rinsed with 13.5 kg of methanol. The solvent was removed under reduced pressure. 13.5kg of isopropanol was added, and then the solvent was removed under reduced pressure again. Recrystallization was carried out by the same recrystallization method as above. The compound 6A was obtained after drying (the compound 6A was characterized as a monohydrate of the compound 6A, and thermogravimetric analysis (TGA) thermogram was shown in FIG 15. The TGA thermogram showed that there was 5.353% weight loss when heating from 25°C to 133°C, corresponding to the loss of the weight of one molecule of water), output: 14.76 kg, water: 5.2%, isopropanol residue: 0.0405%. HPLC purity of compound 6A: 99.94%; HPLC content of the isomer of compound 6A: 0.21%; yield: 66.2%. The $^1$H NMR spectrogram was shown in FIG. 6, the HPLC spectrogram of the compound 6A was shown in FIG. 7, and the HPLC spectrogram of the isomer of the compound 6A was shown in FIG. 8.

Method B

Except for the following differences from the method A, the compound 6A was prepared by the same preparation method as the method A, wherein the amount of the compound 4a was 1 g; the reaction solvent was 1,4-dioxane (6 mL); and R-lactic acid (20 eq) and methanesulfonic acid (2 g) were added. HPLC: 91.1%; HPLC after sodium hydroxide hydrolysis: 98.2%.

Method C

Except for the following differences from the method A, the compound 6A was prepared by the same preparation method as the method A, wherein the amount of the compound 4a was 1 g; the reaction solvent was 1,4-dioxane (10 mL); R-lactic acid (30.2 eq) was added; and the reaction solution was heated for 16 hours at 95-105°C. HPLC: 70%, HPLC after sodium hydroxide hydrolysis: 99.6%; chiral purity: 99.0%.

Method D

Except for the following differences from the method A, the compound 6A was prepared by the same preparation method as the method A, wherein the amount of the compound 4a was 10 g; the reaction solvent was toluene (75 mL); and the reaction solution was heated for 10 hours at 108-113°C. HPLC: 97%; chiral purity: 96%.

**Example 14: Preparation of compound 6C**

R-lactic acid (1.66 g, 5.0 eq), compound 4b (1 g, 1.0 eq) and methylcyclohexane (5.25 g) were added into a reaction flask, and the reaction mixture was refluxed with stirring at 110°C for 24 hours. The reaction was cooled to 20-30°C, and methanol was added. The resulting mixture was allowed to settle for layering. The obtained upper organic phase was discarded, and the obtained lower layer was collected. Sodium hydroxide solution (1.2 g of water, and 0.52 g of sodium hydroxide) was dropwise added with an ice bath and the mixture was stirred for 0.5 hour. Hydrochloric acid (0.23 g of concentrated hydrochloric acid + 2.15 g of water) was dropwise added to adjust the pH to 7. The solution was concentrated under reduced pressure, water was added, the obtained system was stirred for 0.5 hour, and filtered. The

filter cake was dried to obtain the compound 6C (output: 0.984 g, and yield: 82%). The $^1$H NMR spectrogram of the compound 6C was shown in FIG. 14.

**Example 15: Preparation of compound 6B**

Method A

Compound 4a hydrochloride (200 g) and acetic anhydride (600 mL) were combined in a reaction vessel, stirred and heated at 90°C for 12 hours. The reaction solution was cooled to 0-10°C, methyl tert-butyl ether (2 L) was added. The mixture was stirred at 0-10°C for 1 hour, and filtered. The filter cake was rinsed with methyl tert-butyl ether (400 mL), and then placed in water (600 mL) and stirred. Dichloromethane was added. Sodium hydroxide (10% aqueous solution) was added in batches at 15-25°C to adjust the pH to 7-9. After the system was allowed to settle for 0.5 hour, the obtained organic phase was separated, and the obtained aqueous phase was extracted with dichloromethane for three times (600 mL). The organic phases were combined, dried with anhydrous sodium sulfate (200 g), and filtered. The filtrate was concentrated to 400 mL at 30-40°C, and ethanol (400 mL) was added. The resulting solution was concentrated under reduced pressure to 400 mL, and ethanol (400 mL) was added again. The mixture was concentrated under reduced pressure to 600 mL, heated to 45-55°C, stirred until the compound was dissolved to clear. The mixture was cooled to 0-10°C, and water (2L) was slowly added dropwise in about 1 hour. The mixture was stirred at 0-10°C for 1 hour, and filtered. The filter cake was eluted with aqueous solution of ethanol (400 mL, ethanol:water = 1:5), and dried in a vacuum oven at 35-45°C to obtain the compound 6B, i.e., 2-((2R,5S)-5-(2-methyl-1H-furo[3,2-b]imidazo[4, 5-d]pyridine-1-yl)tetrahydro-2H-pyran-2-yl)acetonitrile (output:134.6 g, and yield:89%). $^1$H NMR (400 MHz, Chloroform-*d*) δ 8.96 (s, 1H), 7.90 (d, *J* = 2.3 Hz, 1H), 7.17 (d, *J* = 2.3 Hz, 1H), 4.61 - 4.48 (m, 1H), 4.33 (t, *J* = 11.1 Hz, 1H), 4.11 - 3.99 (m, 2H), 2.78 (dd, *J* = 12.6, 4.2 Hz, 1H), 2.73 (s, 3H), 2.70 (t, *J* = 5.2 Hz, 2H), 2.30 - 2.06 (m, 2H), 1.93 - 1.78 (m, 1H). The $^1$H NMR spectrogram was shown in FIG. 9, and the HPLC spectrogram was shown in FIG. 10.

Method B

Except for the following differences from the method A, the compound 6B was prepared by the same preparation method as the method A, wherein the amount of the compound 4a hydrochloride was 0.5 g; the reagent/solvent was acetic acid (2.5 mL); the temperature was controlled at 100-110°C; reaction time was 23 hours. HPLC: 26%.

Method C

Except for the following differences from the method A, the compound 6B was prepared by the same preparation method as the method A, wherein the amount of the compound 4a hydrochloride was 0.5 g; the reagent/solvent was acetic acid (2.5 mL); sodium acetate (0.24 g, 2 eq) was added; reaction time was 23 hours. HPLC: 93%.

Method D

Except for the following differences from the method A, the compound 6B was prepared by the same preparation method as the method A, wherein acetic acid (0.2 eq) was added to the system; reaction time was 18 hours. HPLC: 99%.

Method E

Except for the following differences from the method A, the compound 6B was prepared by the same preparation method as the method A, wherein sodium acetate (0.5 eq) was added to the system; reaction time was 18 hours. HPLC: 99%.

**Example 16: Preparation of the L-tartrate of compound 6B**

Compound 6B (100 g) and acetone (500 mL) were combined in a reaction vessel, and heated at 40-50°C with stirring for 1 hour until a clear solution was obtained. Acetone (1L) solution containing L-tartaric acid (30 g) was added and the obtained system was stirred for 4 hours at 40-50°C. The solution was cooled to 15-25°C and stirred for 1 hour, then cooled to 0-10°C and stirred for 1 hour. The reaction solution was filtered. The filter cake was eluted with acetone (500 mL), and dried in a vacuum oven at 45-55°C for 24 hours to obtain the L-tartrate of the compound 6B (output: 122.3 g, and yield: 81%). $^1$H NMR (400 MHz, DMSO-$d_6$) δ 8.80 (s, 1H), 8.31 (d, *J* = 2.3 Hz, 1H), 7.24 (d, *J* = 2.2 Hz, 1H), 4.65-4.57 (m, 1H), 4.33 (s, 1H), 4.20 - 4.08 (m, 2H), 3.95 - 3.88 (m, 1H), 3.06 - 2.76 (m, 2H), 2.71 (s, 3H), 2.57 - 2.47 (m, 1H), 2.21 - 2.14 (m, 1H), 2.03-1.97 (m, 1H), 1.81 - 1.66 (m, 1H). The $^1$H NMR spectrogram was shown in FIG. 11, and the HPLC spectrogram was

shown in FIG. 12.

**Example 17: Preparation of cis-trans isomer mixture 8**

Cis-trans isomer mixture 7 (25.1 g, cis:trans = 1:1.7, 1.01 eq), compound 1 (28 g, 1.0 eq), anhydrous ethanol (210 g, 9.5 V) and sodium bicarbonate (54.9 g, 4.5 eq) were combined in a reaction flask, and heated at 70-80°C for 6-10 hours. After the temperature was cooled to room temperature (20-30°C), 20 V of water was dropwise added. The resulting mixture was stirred for 0.5-1.0 hour, stirred for additional 0.5-1.0 hour at 0-10°C. The mixture was filtered, and the filter cake was rinsed with 4 V of water (20-25°C). The filter cake was triturated with 5 V of water at 20-25°C for 0.5-1.0 hour. The mixture was filtered, and rinsed with 4 V of water (20-25°C). The wet product was dried at 50°C to obtain the cis-trans isomer mixture 8 (41.9 g, yield: 98%, purity: 97.9%, and water: 0.26%, wherein the compound 8A: the compound 3a = 1:1.7).

**Example 18: Preparation of cis-trans isomer mixture 9**

Cis-trans isomer mixture **8** (19 g, 1.0 eq), water (76 g, 4 V) and methanol (15.0 g, 1 V) were mixed and heated to 60-70°C. Hypoboric acid (20 g, 1.0 eq + 1.0 eq + 1.0 eq + 0.5 eq) was added in batches, and the reaction mixture was heated at 70-80°C for 1-3 hours. The reaction was stopped when mixture **8** was less than or equal to 1%. The reaction solution was cooled to 45°C and concentrated under reduced pressure to 4-5 V (this process is mainly for removing part of methanol). The reaction solution was cooled to 0-10°C, stirred for 1.0 hour, and filtered under reduced pressure. The filter cake was eluted with 6V of DCM, and then sodium bicarbonate (35.43 g, 0.5 W) was added to the filtrate with stirring. After being stirred for 0.5 -1.0 hour, the aqueous layer was extracted with 3 V of DCM for four times respectively. The obtained organic phases were combined, dried with 1W anhydrous sodium sulfate, and filtered. The filtrate was concentrated to 2 V of DCM, and 10 V of TBME was slowly dropwise added at less than or equal to 30°C. The resulting mixture was mechanically stirred for 1-2 hours, cooled to 1-10°C and kept for 1-2 hours. The mixture was filtered under reduced pressure, and dried at 50°C to obtain the cis-trans isomer mixture 9 (12.77 g, yield: 74.64%, purity: 98.9%, water: 0.79%, and the compound 9A: the compound 4a =1:1.4).

**Example 19: Preparation of mixture 6Aa**

R-lactic acid (13.2 g, 5 eq), cis-trans isomer mixture 9 (8 g, 1 eq), and methylcyclohexane (20 g) were combined and refluxed with a Dean-Stark apparatus until that IPC: isomer intermediate amide was less than 3%. 24 mL of methanol was dropwise added, and the reaction solution was cooled to room temperature. The methanol layer was separated, and 15 mL of methanol was added. The reaction solution was cooled to be less than 0°C, a water solution of sodium hydroxide (4.72 g of sodium hydroxide and 11 g of water) was dropwise added, and the resulting mixture was stirred until the reaction was completed. Dilute hydrochloric acid was dropwise added to adjust the pH to 7-8. The solvents were removed, and 120 mL of water was added. The mixture was filtered. The obtained wet product was put into 120 ml of water, stirred for 2 hours, filtered, and dried at 50°C to obtain 5 g of the solid. The filtrates were combined, extracted with dichloromethane (100 ml*3), and concentrated to obtain 3.5 g of the solid. The solids were combined to obtain the mixture 6Aa (8.5 g, yield: 88.6%, the compound 6Ab:the compound 6A = 1:2).

**Example 20: Preparation of compound 6A**

The mixture 6Aa (6.5 g) and a potassium tert-butoxide solution (0.39 g, 10.5 mL of tetrahydrofuran) were combined, stirred until the isomer 6Ab was less than 2.85%. Diluted hydrochloric acid was added to adjust the pH to 6-7. The solvents were removed by rotary evaporation, and 98 mL of water was added. The resulting mixture was stirred for 2 hours, filtered, and washed with water (20ml*2). The filter cake was collected and dried at 50°C to obtain the compound 6A (5.35 g). 5 g of the compound 6A, 28.3 g of isopropanol, and 12 g of water were refluxed to a clear solution. The reaction solution was cooled at less than 5°C for 3 hours, filtered, washed with a mixed solvent to obtain 4.35 g of wet product. 28.3 g of isopropanol and 12 g of water were added to the wet product, and refluxed to a clear solution. The reaction solution was cooled at less than 5°C for 3 hours, and filtered to obtain the compound 6A (3.5 g, purity: 99.62%, isomer: 0.49%, and yield: 54%). The $^{1}$H NMR spectrogram of the compound 6A obtained was consistent with FIG. 6.

## Claims

**1.** A method for synthesizing 1H-furo[3,2-b]imidazo[4,5-d]pyridine compounds, comprising the following steps of:

step 1:

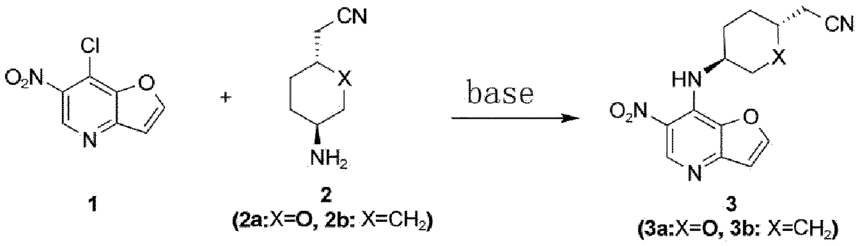


1 2 (2a:X=O, 2b: X=$CH_2$) 3 (3a:X=O, 3b: X=$CH_2$)

reacting compound 1 with compound 2 or compound 2 hydrochloride in a solvent in the presence of a base to obtain compound 3;
step 2:

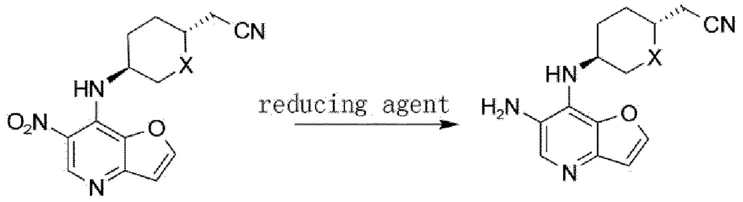


3 (3a:X=O, 3b: X=$CH_2$) 4 (4a:X=O, 4b: X=$CH_2$)

subjecting the compound 3 to a reduction reaction to obtain compound 4 or compound 4 hydrochloride; and
step 3:

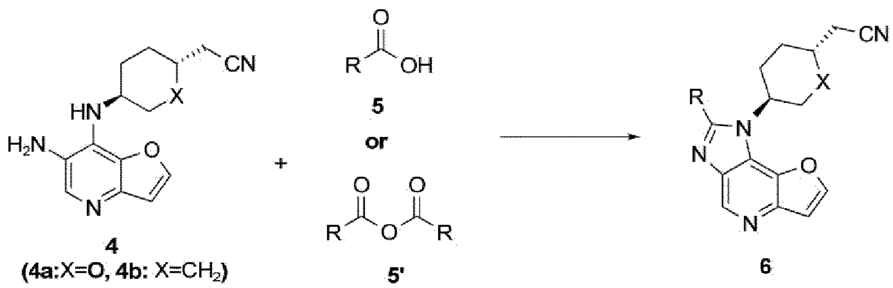

4 (4a:X=O, 4b: X=$CH_2$) 5 or 5' 6

subjecting the compound 4 or compound 4 hydrochloride to a ring-closing reaction with compound 5 or compound 5' to obtain compound 6 or a hydrate of the compound 6, wherein R is methyl or ethyl, the methyl or ethyl is optionally substituted by hydroxyl, and preferably, R is methyl or 1-hydroxyethyl; and
X=$CH_2$, or O.

2. The method according to claim 1, wherein the compound 5 is R-lactic acid, and the step 3 is:

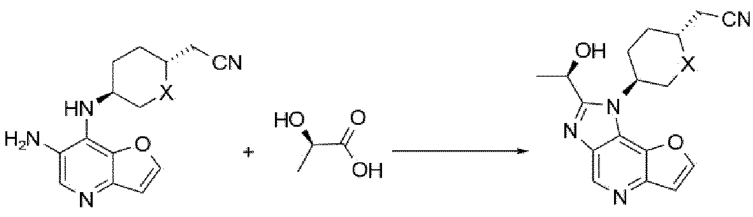

4 (4a:X=O, 4b: X=$CH_2$) 6A(X=O), 6C(X=$CH_2$)

subjecting the compound 4 or compound 4 hydrochloride to a ring-closing reaction with the R-lactic acid to obtain compound 6A or compound 6C, or a hydrate of the compound 6A or the compound 6C.

3. The method according to claim 1, wherein the compound 5' is acetic acid or acetic anhydride, and the step 3 is:

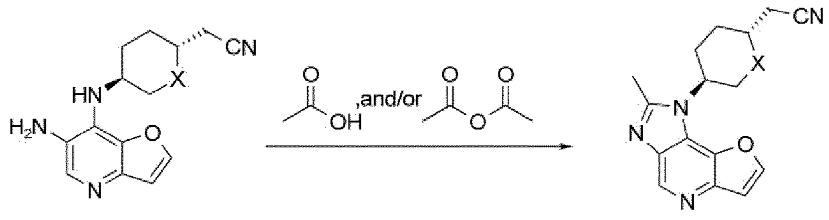


4
(4a:X=O, 4b: X=$CH_2$)

6B(X=O);
6D(X=$CH_2$)

subjecting the compound 4 or compound 4 hydrochloride to a ring-closing reaction with the acetic acid and/or acetic anhydride to obtain compound 6B or compound 6D.

4. The method according to claim 1, wherein in the step 1, the base is an inorganic base.

5. The method according to claim 4, wherein in the step 1, the inorganic base is sodium carbonate, potassium bicarbonate or sodium bicarbonate.

6. The method according to claim 1, wherein in the step 1, the base is an organic base other than N,N-diisopropylethylamine.

7. The method according to claim 6, wherein in the step 1, the organic base is 1,8-diazabicyclo[5.4.0]undec-7-ene (DBU).

8. The method according to claim 1, wherein in the step 1, the base is N,N-diisopropylethylamine, the solvent is a mixed solvent of ethanol and acetonitrile, and preferably, a volume ratio of acetonitrile to ethanol is from 1:0.8 to 1:1.2.

9. The method according to claim 1, wherein in the step 1, the solvent is ethanol and/or acetonitrile, preferably, the solvent is ethanol, or a mixed solvent of acetonitrile and ethanol, wherein a volume ratio of acetonitrile to ethanol is from 1:0.8 to 1:1.2;

the molar ratio of compound 1 to the compound 2 is from 1:0.8 to 1:1.2;
when the compound 2 is in the form of a hydrochloride, the molar ratio of compound 1 to the base is from 1:2 to 1:2.5, and preferably from 1:2.1 to 1:2.3;
when the compound 2 is in a salt-free form, the molar ratio of the compound 1 to the base is from 1:1 to 1:1.5, and preferably from 1:1.1 to 1:1.3;
the mass-to-volume ratio of compound 1 to the solvent is from 1:9 to 1:10;
the reaction of the step 1 is optionally carried out under the protection of an inert gas;
the reaction is carried out at a temperature of 50-80°C; and
after the reaction is completed, the reaction solution is cooled, stirred and filtered, and the filter cake is washed and dried to obtain the compound 3.

10. The method according to any one of claims 1 to 9, wherein the reducing agent in the step 2 is a nitro reducing agent other than $H_2$/Pd;

the reaction of the step 2 is optionally carried out under the protection of an inert gas; and
the reaction is carried out at a temperature of 15-90°C.

11. The method according to claim 10, wherein in the step 2, the reducing agent is Fe, $B_2(OH)_4$, or $SnCl_2$.

12. The method according to claim 11, wherein in the step 2, when the reducing agent is Fe,

the solvent used in the reduction reaction is acetonitrile; the molar ratio of compound 3 to Fe is from 1:6 to 1:9, and the molar ratio of compound 3 to the acetic acid is from 1:12 to 1:14; and the mass-to-volume ratio of compound 3 to the solvent is from 1:4 to 1:10, and preferably from 1:5 to 1:9; and
after the reaction is completed, the reaction solution is filtered, the pH is adjusted to about 6 with sodium citrate solution, and then adjusted to 8-9 with dilute $K_3PO_4$ solution; after concentration, celite, water and ethyl acetate

are added to the reaction solution, and the solution is filtered and layered; the obtained aqueous phase is extracted with ethyl acetate, and the obtained organic phases are combined, washed with sodium citrate aqueous solution, and brine respectively; silica gel and anhydrous sodium sulfate are added, and the solution is filtered, washed with ethyl acetate, and concentrated; methyl tert-butyl ether is added , and the precipitate is filtered and dried to obtain the compound 4.

**13.** The method according to claim 11, wherein in the step 2, when the reducing agent is $B_2(OH)_4$,

the solvent used in the reduction reaction is a mixed solvent of water and methanol, wherein a volume ratio of water to methanol is from 1:1 to 9:1, preferably from 3:1 to 5:1, more preferably 4:1; the molar ratio of compound 3 to $B_2(OH)_4$ is from 1:3 to 1:5, and preferably 1:3.5; the mass-to-volume ratio of the compound 3 to the solvent is from 1:4 to 1:10, and preferably from 1:5 to 1:9; and $B_2(OH)_4$ is added in batches;
4,4'-bipyridine is optionally added; and
after the reaction is completed, the reaction solution is filtered, and the filter cake is washed with dichloromethane; the filtrate is collected and sodium bicarbonate is added; the obtained system is stirred evenly, and is allowed to settle; the organic phase is separated, and the aqueous phase is extracted with dichloromethane; then the organic phases are combined, dried with anhydrous sodium sulfate, and hydrochloric acid in ethanol solution is dropwise added; the obtained system is stirred evenly, and ethyl acetate is dropwise added; the obtained system is stirred evenly, and filtered, then the filter cake is rinsed with ethyl acetate, and dried to obtain the compound 4 hydrochloride.

**14.** The method according to claim 11, wherein in the step 2, when the reducing agent is stannous chloride $SnCl_2$,

the solvent used in the reduction reaction is ethyl acetate; the molar ratio of compound 3 to the stannous chloride is from 1:2 to 1:9, and preferably from 1:3 to 1:6; and the mass-to-volume ratio of compound 3 to the solvent is from 1:4 to 1:20, and preferably from 1:5 to 1:15; and
after the reaction is completed, sodium bicarbonate is added, and the obtained system is stirred, filtered, and rinsed with ethyl acetate; then the filtrate is collected, and washed with aqueous solution of saturated sodium bicarbonate; the obtained organic phases are combined and then washed with brine, dried with anhydrous sodium sulfate, and filtered; the filtrate is concentrated under reduced pressure to obtain the compound 4.

**15.** The method according to claim 2 or 3, wherein in the step 3,

the ring-closing reaction is carried out in a solvent, wherein the solvent is toluene, dioxane, acetic acid, methylcyclohexane or acetic anhydride;
when the compound 4 is subjected to the ring-closing reaction with R-lactic acid, the molar ratio of compound 4 to the R-lactic acid is from 1:4 to 1:30, preferably from 1:4 to 1:20, and more preferably from 1:4 to 1:10; and the mass-to-volume ratio of compound 4 to the solvent is from 1:1.5 to 1:40, preferably from 1:1.5 to 1:30, and more preferably from 1:1.5 to 1:15;
when the compound 4 is subjected to the ring-closing reaction with acetic anhydride or acetic acid, the acetic anhydride or the acetic acid act as a reaction reagent and a solvent simultaneously, and the mass-to-volume ratio of compound 4 to the acetic anhydride or the acetic acid is from 1:1 to 1:10, and preferably from 1:2 to 1:5;
an acid catalyst is optionally added in the ring-closing reaction of the compound 4 with the compound 5 or the compound 5', wherein the acid catalyst is preferably methanesulfonic acid;
the reaction is carried out at a temperature of 80-120°C; and
after the reaction is completed, the reaction solution is cooled and extracted, the pH is adjusted to 7-10, and the obtained solid is dried to obtain the compound 6 or the hydrate of the compound 6.

**16.** The method according to claim 2, wherein in the method for synthesizing the compound 6A, in the step 3, the compound 4 is compound 4a,

the ring-closing reaction is carried out in a solvent, wherein the solvent is toluene, dioxane, or methylcyclohexane, preferably toluene or methylcyclohexane, and more preferably methylcyclohexane;
the molar ratio of compound 4a to the R-lactic acid is from 1:4 to 1:30, preferably from 1:4 to 1:20, more preferably from 1:4 to 1:10, and most preferably from 1:5 to 1:7;
a mass-to-volume ratio (g/mL) of the compound 4a to the solvent is from 1:1.5 to 1:40, preferably from 1:1.5 to 1:30, more preferably from 1:1.5 to 1:15, and most preferably from 1:1.5 to 1:5; and
after the reaction is completed, methanol is added, the methanol layer is separated; sodium hydroxide solution is

added with stirring, subsequently hydrochloric acid is dropwise added to adjust the pH to about 7; the solvent is removed under reduced pressure, and water is added with stirring; the solid is filtered, washed with water, and dried to obtain the compound 6A.

**17.** The method according to claim 2, wherein in the method for synthesizing the compound 6C, in the step 3, the compound 4 is compound 4b,

the ring-closing reaction is carried out in a solvent, wherein the solvent is toluene, dioxane, or methylcyclohexane, preferably toluene or methylcyclohexane, and more preferably methylcyclohexane;
the molar ratio of compound 4b to the R-lactic acid is from 1:4 to 1:30, preferably from 1:4 to 1:20, more preferably from 1:4 to 1:10, and most preferably from 1:5 to 1:7;
a mass-to-volume ratio (g/mL) of the compound 4b to the solvent is from 1:1.5 to 1:40, preferably from 1:1.5 to 1:30, more preferably from 1:1.5 to 1:15, and most preferably from 1:1.5 to 1:5; and
after the reaction is completed, methanol is added, and the methanol layer is separated; sodium hydroxide solution is added with stirring, subsequently hydrochloric acid is dropwise added to adjust the pH to about 7; the solvent is removed under reduced pressure, and water is added with stirring; the solid is filtered, washed with water, and dried to obtain the compound 6C.

**18.** The method according to claim 3, wherein in the method for synthesizing the compound 6B, in the step 3, the compound 4 is compound 4a,

when the compound 4a is subjected to the ring-closing reaction with acetic anhydride, acetic acid or sodium acetate is optionally added, wherein the molar ratio of compound 4a to the acetic acid is from 1:0.1 to 1:0.5, and preferably from 1:0.2 to 1:0.3, and the molar ratio of compound 4a to the sodium acetate is from 1:0.2 to 1:1; and
when the compound 4a is subjected to the ring-closing reaction with acetic acid, sodium acetate is optionally added, wherein the molar ratio of compound 4a to the sodium acetate is from 1:1 to 1:3.

**19.** The method according to claim 3, wherein in the method for synthesizing the compound 6B, in the step 3, the compound 4 is compound 4a,

when the compound 4a is subjected to the ring-closing reaction with acetic anhydride, the mass-to-volume ratio of compound 4a to the acetic anhydride is from 1:2 to 1:5;
when the compound 4a is subjected to the ring-closing reaction with acetic acid, the mass-to-volume ratio of compound 4a to the acetic acid is from 1:3 to 1:8.
the reaction is carried out at a temperature of 80-120°C, and preferably 90-110°C; and
after the reaction is completed, methyl tert-butyl ether is added, the obtained system is stirred evenly, and filtered; the filter cake is dissolved in a mixed solvent of water and dichloromethane, and NaOH aqueous solution is dropwise added to adjust the pH to 7-10; then the reaction solution is layered, and the obtained aqueous layer is extracted with dichloromethane; the obtained organic phases are combined, dried with anhydrous $Na_2SO_4$, filtered, and concentrated; ethanol and water are added with stirring, and filtered, then the filter cake is dried to obtain the compound 6B.

**20.** The method according to claim 3, wherein in the method for synthesizing the compound 6D, in the step 3, the compound 4 is compound 4b,

when the compound 4b is subjected to the ring-closing reaction with acetic anhydride, acetic acid or sodium acetate is optionally added, wherein the molar ratio of compound 4b to the acetic acid is from 1:0.1 to 1:0.5, and preferably from 1:0.2 to 1:0.3; and the molar ratio of compound 4b to the sodium acetate is from 1:0.2 to 1:1, and preferably from 1:0.4 to 1:0.8; and
when the compound 4b is subjected to the ring-closing reaction with acetic acid, sodium acetate is optionally added, wherein the molar ratio of compound 4b to the sodium acetate is from 1:1 to 1:3, and preferably from 1:1.5 to 1:2.5.

**21.** The method according to claim 3, wherein in the method for synthesizing the compound 6D, in the step 3, the compound 4 is compound 4b,

when the compound 4b is subjected to the ring-closing reaction with acetic anhydride, the mass-to-volume ratio of compound 4b to the acetic anhydride is from 1:2 to 1:5;

when the compound 4b is subjected to the ring-closing reaction with acetic acid, the mass-to-volume ratio of compound 4b to the acetic acid is from 1:3 to 1:8;
the reaction is carried out at a temperature of 80°C - 120°C, and preferably 90°C - 110°C; and
after the reaction is completed, methyl tert-butyl ether is added, the obtained system is stirred evenly, and filtered; the filter cake is dissolved in a mixed solvent of water and dichloromethane, and NaOH aqueous solution is dropwise added to adjust the pH to 7-10; then the reaction solution is layered, and the obtained aqueous layer is extracted with dichloromethane; the obtained organic phases are combined, dried with anhydrous $Na_2SO_4$, filtered, and concentrated; ethanol and water are added with stirring, and filtered, then the filter cake is dried to obtain the compound 6D.

**22.** The method according to claim 1, wherein the compound 1 is synthesized by the following steps of:

step 1-1:

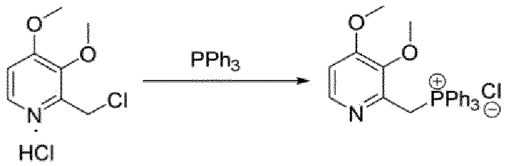


1-1 1-2

reacting compound 1-1 with triphenylphosphine to obtain compound 1-2;
step 1-2:

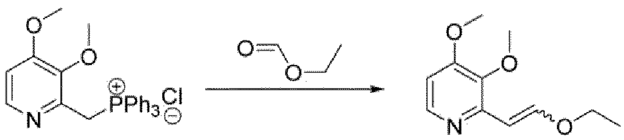

1-2 1-3

reacting the compound 1-2 with ethyl formate to obtain compound 1-3;
step 1-3:

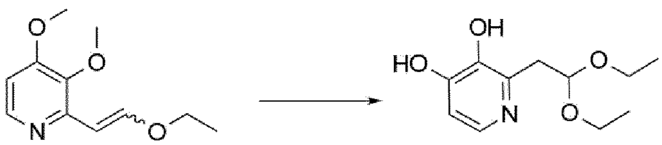

1-3 1-4

subjecting the compound 1-3 to a demethylation and acetalization reaction to obtain compound 1-4;
step 1-4:

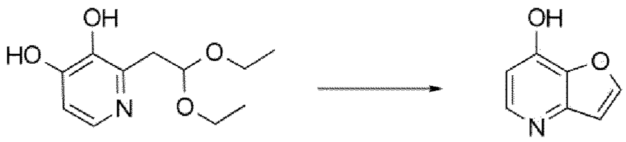

1-4 1-5

subjecting the compound 1-4 to a ring-closing reaction under acidic condition to obtain compound 1-5;
step 1-5:

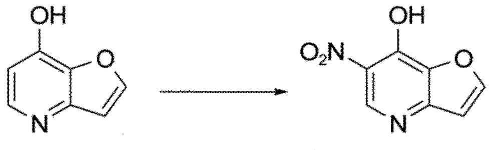

1-5 1-6

subjecting the compound 1-5 to a nitration reaction to obtain compound 1-6; and step 1-6:

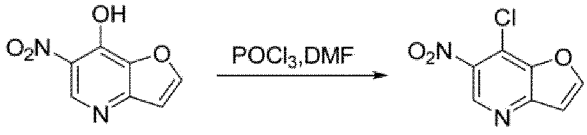


1-6 1

subjecting the compound 1-6 to a chloro-substitution reaction to obtain the compound 1.

**23.** The method according to claim 22, wherein in the method for synthesizing the compound 1,

in the step 1-1:

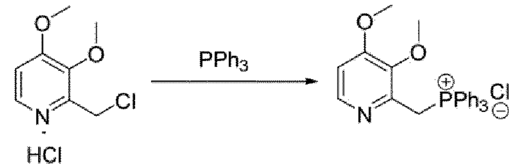


1-1 1-2

sodium carbonate is added to a mixture of the compound 1-1, toluene and water; the obtained system is stirred at room temperature, and layered; the obtained aqueous phase is extracted with toluene, and the obtained organic phases are washed with brine, dried with anhydrous sodium sulfate, and filtered; triphenylphosphine is added to the filtrate, and the obtained system is heated at 110-120°C with a Dean-Stark apparatus; then the reaction solution is cooled to 20-30°C after the reaction is completed, and filtered; the obtained wet product is dried under reduced pressure to obtain the compound 1-2;
in the step 1-2:

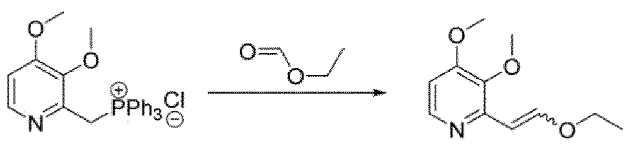

1-2 1-3

alkali metal tert-butoxide is added to a mixture of the compound 1-2 and dimethyl sulphoxide, and the obtained system is stirred evenly at room temperature; ethyl formate is dropwise added, and the reaction solution is stirred at 40-50°C; then the reaction solution is cooled to room temperature after the reaction is completed; the reaction solution is added into an organic solvent and water for extraction, and the obtained organic phases are washed with water, adjusted the pH to 5-6 with hydrochloric acid, and layered; the obtained aqueous phase is extracted with an organic solvent, and the pH is adjusted to be greater than or equal to 8 with a base; then the system is layered, and the obtained aqueous phase is extracted with an organic solvent; the obtained organic phases are combined, washed with water, dried with anhydrous sodium sulfate, filtered, and concentrated to obtain the compound 1-3;
in the step 1-3:

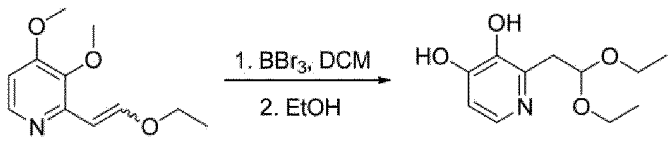


1-3 1-4

boron tribromide is dropwise added into a mixture of the compound 1-3 and dichloromethane, and the reaction solution is stirred at 20-30°C after the dropwise addition; the reaction solution is dropwise added into ethanol, stirred evenly, and concentrated under reduced pressure; After ethanol is added, the solution is concentrated under reduced pressure, adjusted the pH to 5-6 with dropwise addition of an ethanol solution of sodium ethoxide, filtered, and the filter cake is rinsed with ethanol; an ethanol solution of sodium ethoxide is dropwise added to the filtrate to adjust the pH to 8-9, and the solvent is removed under reduced pressure; methyl tert-butyl ether is added, and the obtained system is stirred evenly, filtered, and then the filter cake is rinsed with methyl tert-butyl ether, and dried to obtain the compound 1-4;
in the step 1-4:

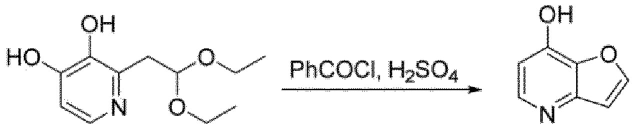


1-4 1-5

benzoyl chloride is dropwise added to a dichloromethane solution of the compound 1-4, and the obtained system is stirred evenly after the dropwise addition; the reaction solution is dropwise added into concentrated sulfuric acid, and the obtained system is stirred evenly, then the reaction solution is dropwise added into water; the obtained system is filtered, and the pH of the filtrate is adjusted to 7-8; the reaction solution is stirred evenly after the dropwise addition, filtered, and then the filter cake is rinsed with water, and dried to obtain the compound 1-5;
in the step 1-5:

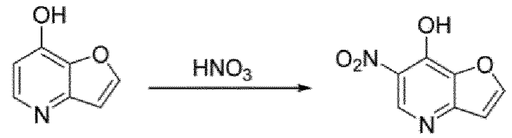


1-5 1-6

nitric acid is dropwise added to a mixed solution of acetic acid and acetic anhydride, the resulting mixture is stirred evenly after the dropwise addition; the mixed solution of acetic acid, acetic anhydride and nitric acid is dropwise added into an acetic acid solution of the compound 1-5; the obtained system is stirred evenly after the dropwise addition, then methyl tert-butyl ether is added to the reaction solution after the reaction is completed; the obtained system is stirred uniformly and filtered, and then the filter cake is rinsed with methyl tert-butyl ether, and dried to obtain the compound 1-6; and
in the step 1-6:

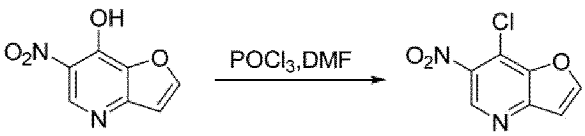


1-6 1

phosphorus oxychloride is dropwise added to a mixture of the compound 1-6, N,N-dimethylformamide and toluene; the obtained system is stirred at 35-75°C, and the reaction solution is cooled to room temperature after

the reaction is completed; toluene is removed, and dichloromethane and potassium carbonate aqueous solution or sodium bicarbonate aqueous solution are added; the system is allowed to be layered; the obtained organic phases are washed with brine, dried with anhydrous sodium sulfate, filtered, and concentrated to obtain the compound 1.

**24.** The method according to claim 22, wherein in the step 1-1,

in the mixture of the compound 1-1, toluene and water, the mass-to-volume ratio of compound 1-1 to toluene and water is from 1:5 to 1:6, and a volume ratio of toluene to water is from 8:1 to 9:1;
the molar ratio of compound 1-1 to sodium carbonate is from 1:1.1 to 1:1.2;
the molar ratio of compound 1-1 to triphenylphosphine is from 1:1.1 to 1:1.2; and
the reaction of the step 1-1 is optionally carried out under the protection of an inert gas.

**25.** The method according to claim 22, wherein in the step 1-2,

the alkali metal tert-butoxide is potassium tert-butoxide or sodium tert-butoxide;
the base used for adjusting the pH is aqueous ammonia or sodium carbonate;
the organic solvent used for extraction is methyl tert-butyl ether or ethyl acetate;
the mass-to-volume ratio of compound 1-2 to dimethyl sulphoxide is from 1:3 to 1:5;
the molar ratio of compound 1-2 to alkali metal tert-butoxide is from 1:1.05 to 1:1.15, and preferably 1:1.1;
the molar ratio of compound 1-2 to ethyl formate is from 1:4 to 1:5; and
the reaction of the step 1-2 is optionally carried out under the protection of an inert gas.

**26.** The method according to claim 22, wherein in the step 1-3,

the mass-to-volume ratio of compound 1-3 to dichloromethane is from 1:6 to 1:10;
the molar ratio of compound 1-3 to boron tribromide is from 1:2.0 to 1:3.5, and preferably from 1:2.5 to 1:3.2; and
the reaction of the step 1-3 is optionally carried out under the protection of an inert gas.

**27.** The method according to claim 22, wherein in the step 1-4,

the mass-to-volume ratio of compound 1-4 to concentrated sulfuric acid is from 1:2 to 1:5;
the mass-to-volume ratio of compound 1-4 to dichloromethane is from 1:25 to 1:26; and
the molar ratio of compound 1-4 to benzoyl chloride is from 1:1.5 to 1:2.5, and preferably 1:2.

**28.** The method according to claim 22, wherein in the step 1-5,

the mass ratio of the compound 1-5 to the acetic acid in the acetic acid solution of the compound 1-5 is from 1:9 to 1:11, and preferably 1:10;
the mass ratio of compound 1-5 to the acetic acid in the mixed solution of acetic acid, acetic anhydride and nitric acid is from 1:3 to 1:5;
the molar ratio of compound 1-5 to acetic anhydride is from 1:2 to 1:5, and preferably from 1:2.6 to 1:4.2;
the molar ratio of compound 1-5 to nitric acid is from 1:2 to 1:5, and preferably from 1:2.5 to 1:4.0; and
the reaction of the step 1-5 is optionally carried out under the protection of an inert gas.

**29.** The method according to claim 22, wherein in the step 1-6,

the molar ratio of compound 1-6 to N,N-dimethylformamide is from 1:1 to 1:1.2;
the mass-to-volume ratio of compound 1-6 to toluene is from 1:9 to 1:11, and preferably 1:10;
the molar ratio of compound 1-6 to phosphorus oxychloride is from 1:1.8 to 1:2.2, and preferably 1:2; and
the reaction of the step 1-6 is optionally carried out under the protection of an inert gas.

**30.** The method according to claim 1, wherein when the compound 2 is a compound 2a, the compound 2a hydrochloride is synthesized through the following steps of:

step 2-1:

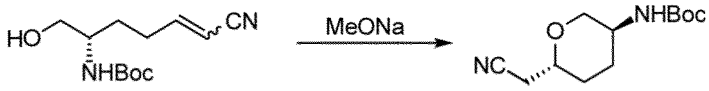


2-1 2-2

subjecting compound 2-1 to a ring-closing reaction under the catalysis of sodium methoxide to obtain compound 2-2; and

step 2-2:

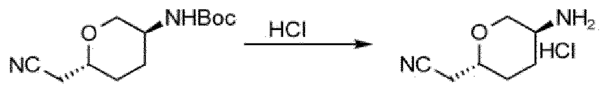


2-2 2a

reacting the compound 2-2 with hydrochloric acid to obtain the compound 2a hydrochloride.

**31.** The method according to claim 30, wherein in the method for synthesizing the compound 2a hydrochloride,

in the step 2-1:

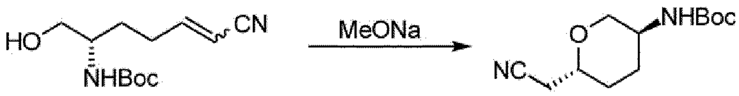


2-1 2-2

a methanol solution of sodium methoxide is dropwise added to a mixture of the compound 2-1 and tetrahydrofuran, and the obtained system is stirred evenly after the dropwise addition; after the reaction is completed, ammonium chloride solution is dropwise added to the reaction solution to adjust the pH to 7-8; the mixture is extracted with methyl tert-butyl ether, washed with sodium chloride solution, dried with anhydrous sodium sulfate, filtered, and dried to obtain the compound 2-2; and

in the step 2-2:

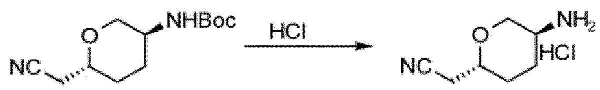


2-2 2a

an ethanol solution of hydrogen chloride is dropwise added to a mixture of the compound 2-2 and dichloromethane; the obtained system is stirred evenly after the dropwise addition, and filtered after the reaction is completed; the filter cake is rinsed with dichloromethane, and dried to obtain the compound 2a hydrochloride.

**32.** The method according to claim 30, wherein in the step 2-1,

the mass-to-volume ratio of compound 2-1 to tetrahydrofuran is from 1:10 to 1:12, and preferably from 1:10 to 1:11;

the molar ratio of compound 2-1 to sodium methoxide in methanol solution is from 1:0.2 to 1:0.3, and preferably 1:0.25; and

the reaction of the step 2-1 is optionally carried out under the protection of an inert gas.

**33.** The method according to claim 30, wherein in the step 2-2,

the mass-to-volume ratio of compound 2-2 to dichloromethane is from 1:5 to 1:7, and preferably 1:6;

the molar ratio of compound 2-2 to hydrogen chloride in ethanol solution is from 1:2 to 1:5, and preferably 1:3; and

the reaction of the step 2-2 is optionally carried out under the protection of an inert gas.

**34.** A method for synthesizing 1H-furo[3,2-b]imidazo[4,5-d]pyridine compounds, comprising the following steps of:

step 1A:

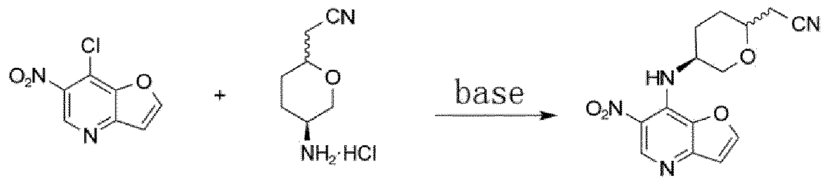


1 7 8

reacting compound 1 with cis-trans isomer mixture 7 in a solvent in the presence of a base to obtain cis-trans isomer mixture 8;

step 2A:

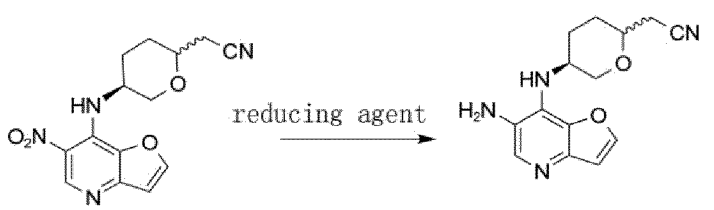


8 9

subjecting the cis-trans isomer mixture 8 to a reduction reaction to obtain cis-trans isomer mixture 9 or the cis-trans isomer mixture 9 hydrochlorides;

step 3A:

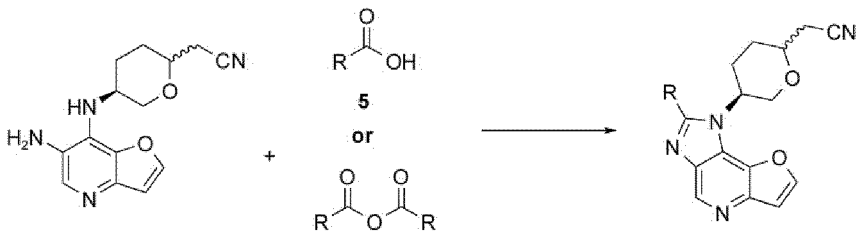

5

or

9 5' 10

subjecting the cis-trans isomer mixture 9 or the cis-trans isomer mixture 9 hydrochlorides to a ring-closing reaction with compound 5 or compound 5' to obtain mixture 10 or hydrates of the mixture 10, wherein R is methyl or ethyl, the methyl or ethyl is optionally substituted by hydroxyl, and preferably, R is methyl or 1-hydroxyethyl; and

step 4A:

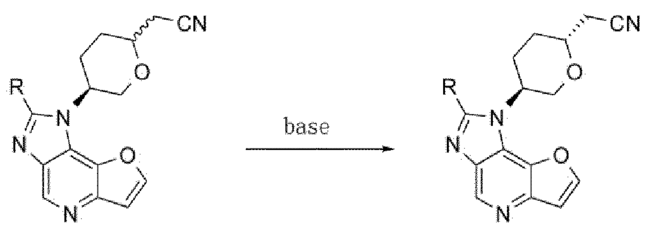


10 6

subjecting the mixture 10 or the hydrates of the mixture 10 to an isomerization reaction under basic condition to obtain compound 6 or a hydrate of the compound 6.

**35.** The method according to claim 34, wherein the reaction condition of the step 1A are as defined in any one of claims 4 to 9.

**36.** The method according to claim 34, wherein the reaction condition of the step 2A are as defined in any one of claims 10 to 14.

**37.** The method according to claim 34, wherein the reaction condition of the step 3A are as defined in any one of claims 2, 3 and 15 to 21.

**38.** The method according to claim 34, wherein the step 4A is:

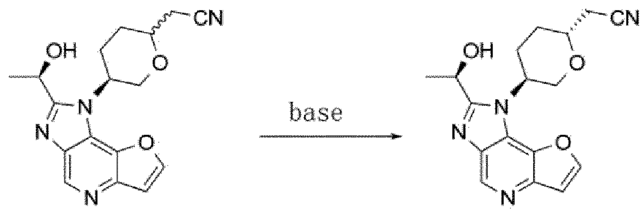


6Aa 6A

subjecting mixture 6Aa or hydrates of the mixture 6Aa to an isomer transformation reaction under basic condition to obtain a hydrate of compound 6A.

**39.** The method according to claim 38, wherein the base is an alkoxide base, preferably alkali metal $C_{1-6}$ alkoxide, and more preferably, potassium tert-butoxide.

**40.** The method according to claims 38 to 39, wherein in the method for synthesizing the hydrate of the compound 6A, in the step 4A,

the reaction is carried out in a solvent, wherein the solvent is preferably tetrahydrofuran;
the molar ratio of mixture 6Aa to the base is from 1:0.05 to 1:0.2, and preferably from 1:0.1 to 1:0.2;
the reaction is carried out at room temperature; and
after the reaction is completed, dilute hydrochloric acid is added to adjust the pH to 6-7; the solvent is removed by rotary evaporation, and water is added with stirring; the mixture is filtered, and washed with water; the filter cake is collected and dried to obtain the hydrate form of the compound 6A.

**41.** The method according to claim 34, wherein the step 4A is:

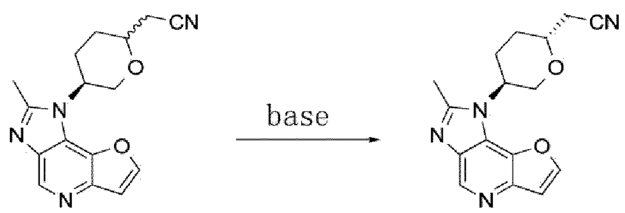


6Ba 6B

subjecting mixture 6Ba or the mixture 6Ba hydrochlorides to an isomer transformation reaction under basic condition to obtain compound 6B.

**42.** The method according to claim 41, wherein the base is an alkoxide base, preferably alkali metal $C_{1-6}$ alkoxide, and more preferably, potassium tert-butoxide.

**43.** The method according to claims 41 to 42, wherein in the method for synthesizing the compound 6B, in the step 4A,

the reaction is carried out in a solvent, wherein the solvent is preferably tetrahydrofuran;
the molar ratio of mixture 6Ba to the base is from 1:0.05 to 1:0.2, and preferably from 1:0.1 to 1:0.2;
the reaction is carried out at room temperature; and
after the reaction is completed, dilute hydrochloric acid is added to adjust the pH to 6-7; the solvent is removed by rotary evaporation, and water is added with stirring; the mixture is filtered, and washed with water; the filter cake is

collected and dried to obtain the compound 6B.

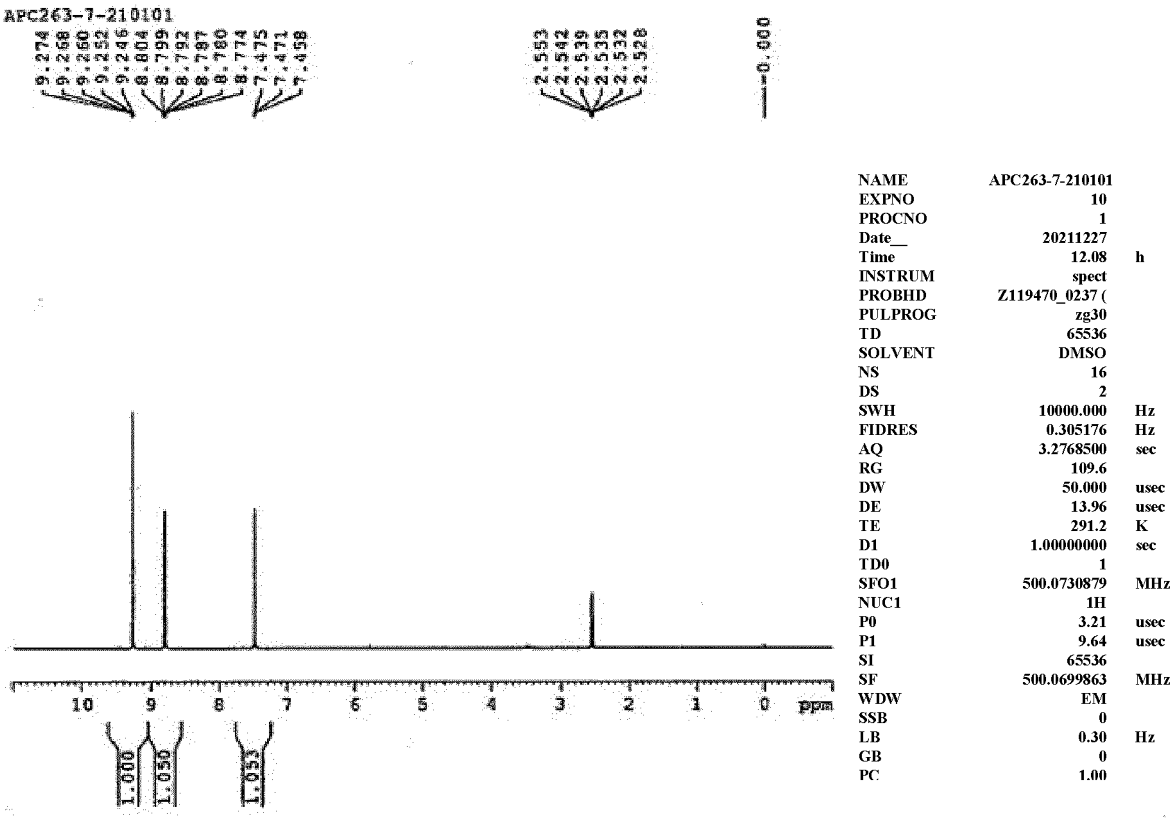


Fig. 1

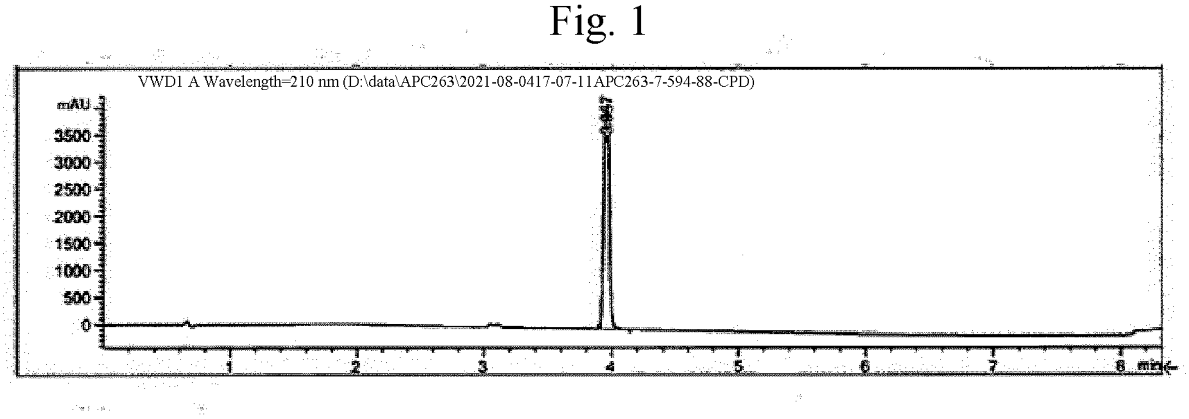


| Retention time (ms) | MS area | Molecular weight or ion |
|---|---|---|
| 4.040 | 1911831 | 201.00 I |
| | | 199.00 I |

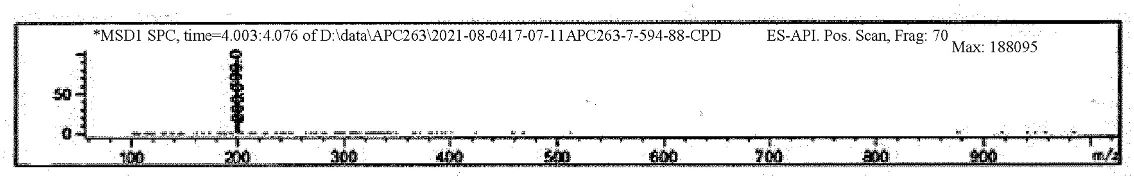


Fig. 2

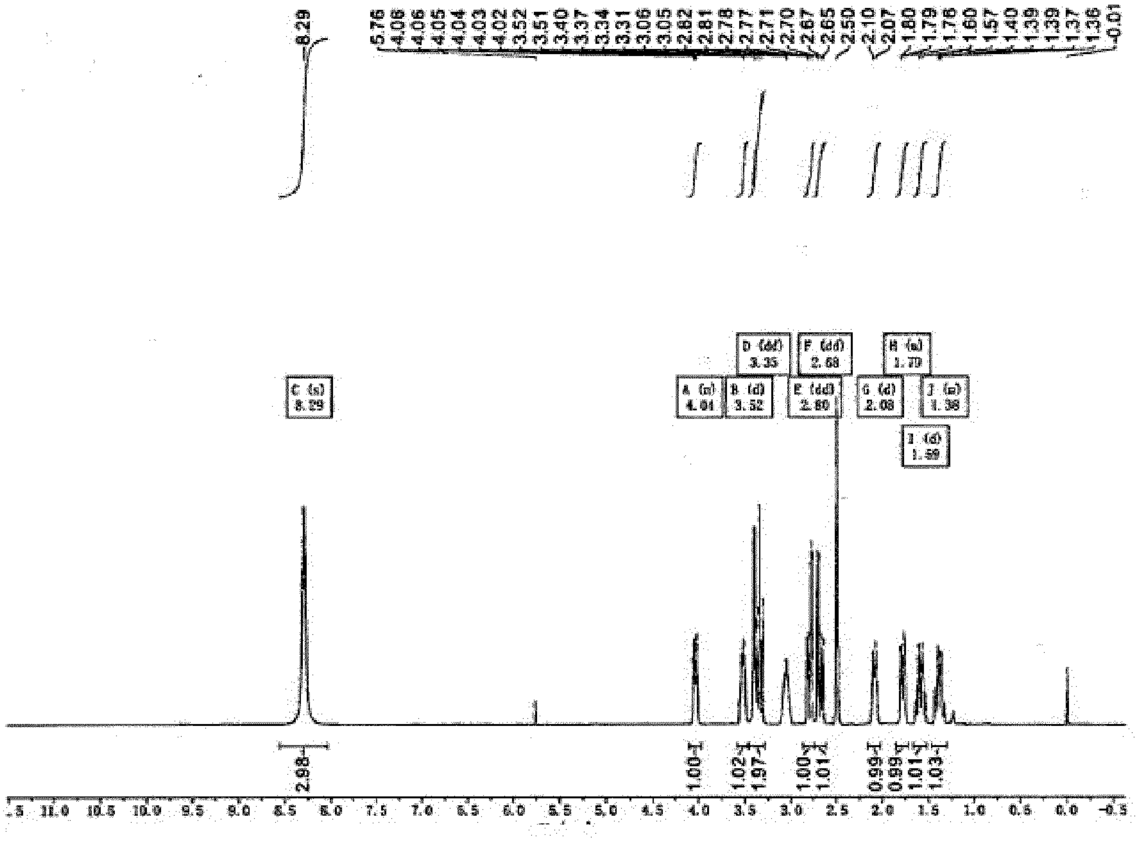

Fig. 3

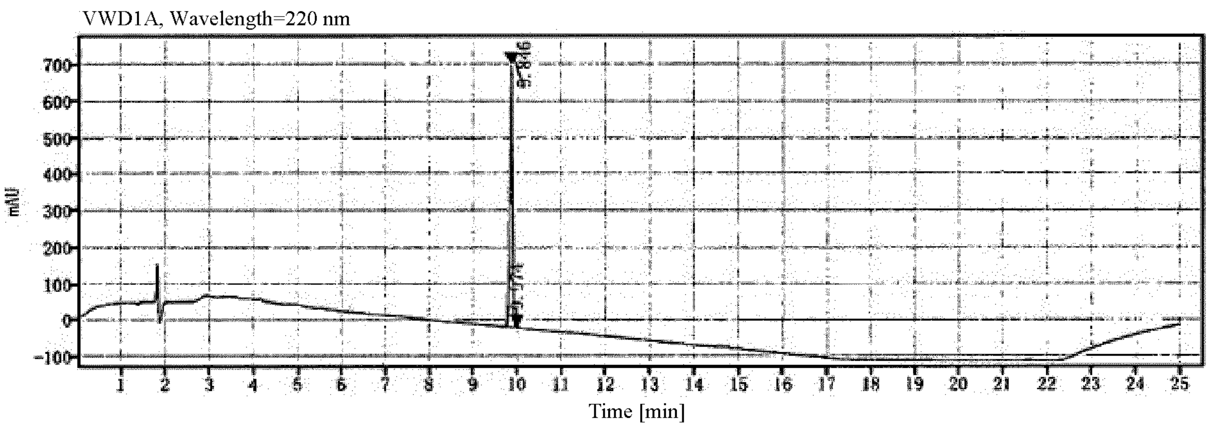


**Signal:** **VWD1, Wavelength=220 nm**

| Retention time [min] | Type | Peak Width [min] | Peak Area | Peak Height | Peak area% | Name |
|---|---|---|---|---|---|---|
| 9.846 | BM m | 0.29 | 2898.76 | 718.50 | 99.83 | |
| 9.974 | MM m | 0.09 | 5.00 | 1.65 | 0.17 | |
| | | Sum | 2903.76 | | | |

Fig. 4

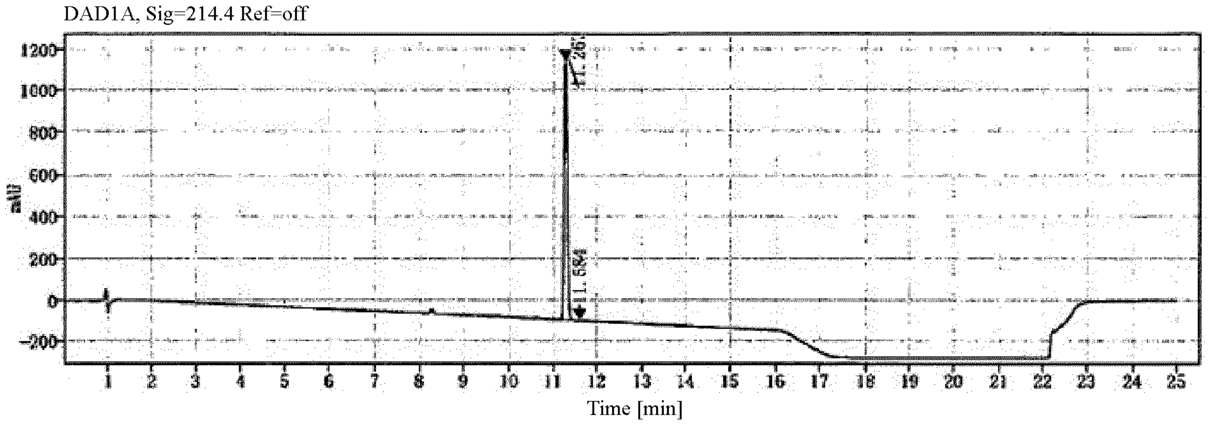


**Signal:** **DAD1A, Sig=214.4 Ref=off**

| Retention time [min] | Type | Peak width [min] | P area | Peak height | Peak area% | Name |
|---|---|---|---|---|---|---|
| 11.267 | BM m | 0.07 | 5732.36 | 1233.46 | 99.96 | |
| 11.584 | MM m | 0.07 | 2.53 | 0.58 | 0.04 | |
| | | Sum | 5734.89 | | | |

Fig. 5

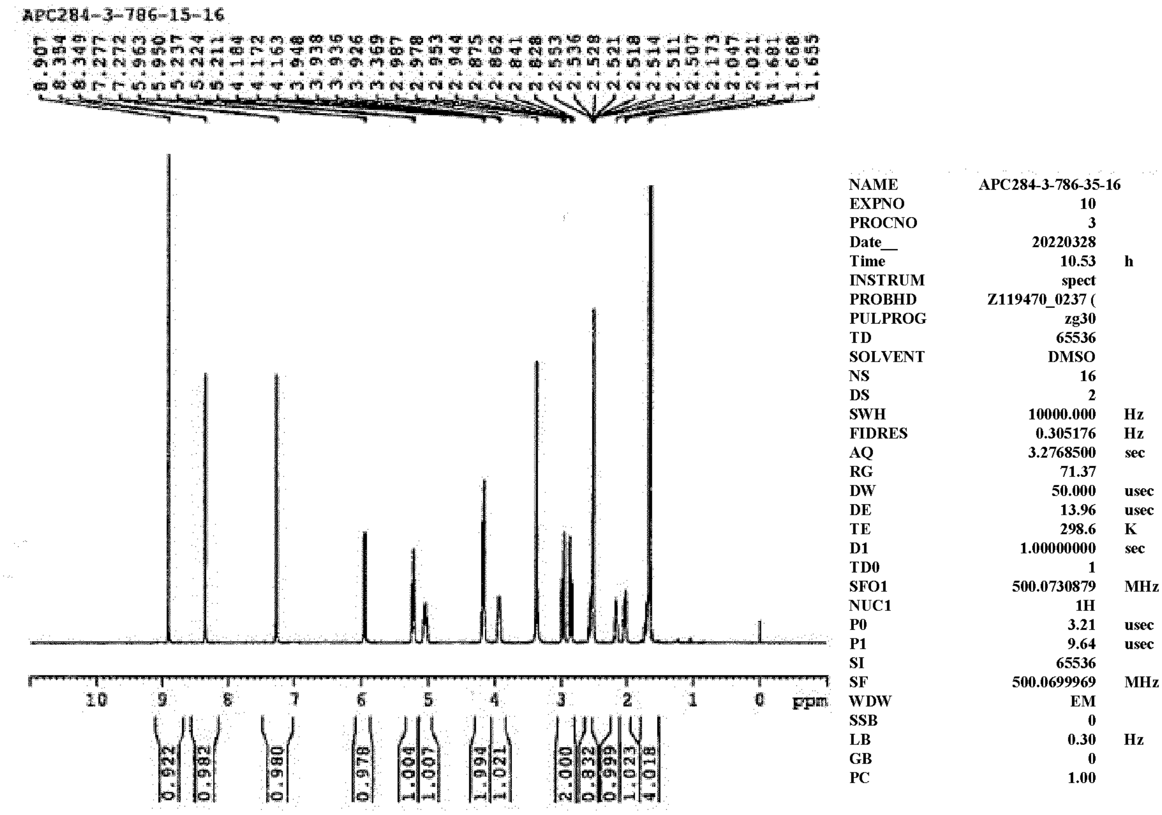


Fig. 6

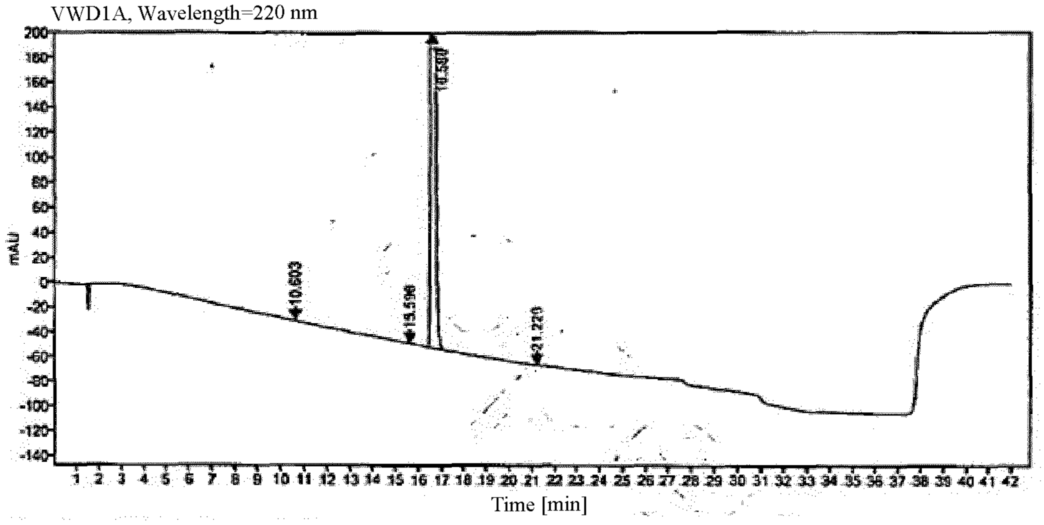


Signal: VWD1A, Wavelength=220 nm

| Peak # | Compound Name | RT [min] | Area [mAU*s] | Height [mAU] | Area% | Plates | Resolution | Tail Factor | S/N |
|---|---|---|---|---|---|---|---|---|---|
| 1 | | 10.603 | 3.841 | 0.524 | 0.02 | 54789 | | 0.94 | |
| 2 | | 15.596 | 1.956 | 0.199 | 0.01 | 111866 | 27.15 | 1.20 | |
| 3 | | 16.586 | 15976.478 | 1673.804 | 99.94 | 69275 | 4.51 | 1.43 | |
| 4 | | 21.229 | 3.654 | 0.344 | 0.02 | 107102 | 18.15 | 1.03 | |
| | | Sum | 15985.928 | | | | | | |

Fig. 7

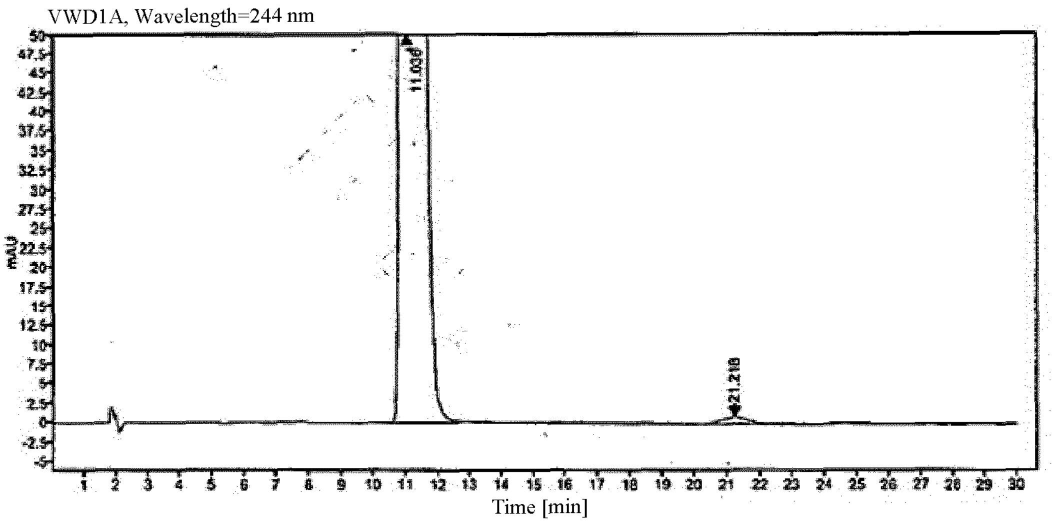


Signal: VWD1A, Wavelength=244 nm

| Peak # | Compound Name | RT [min] | Area [mAU*s] | Height [mAU] | Area% | Plates | Resolution | Tail Factor | S/N |
|---|---|---|---|---|---|---|---|---|---|
| 1 | | 11.036 | 20086.443 | 703.721 | 99.79 | 3266 | | 1.81 | |
| 2 | | 21.218 | 42.735 | 0.823 | 0.21 | 3717 | 9.41 | 0.97 | |
| | | Sum | 20129.178 | | | | | | |

Fig. 8

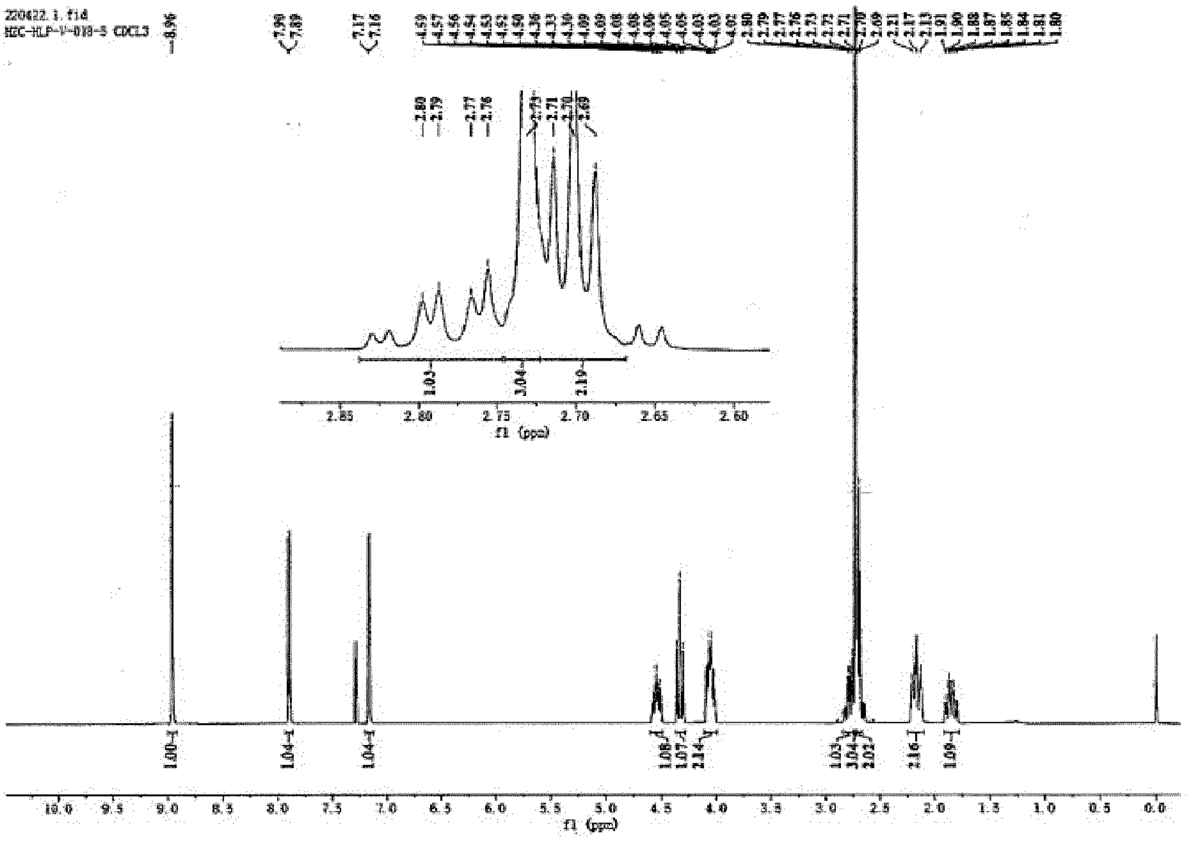


Fig. 9

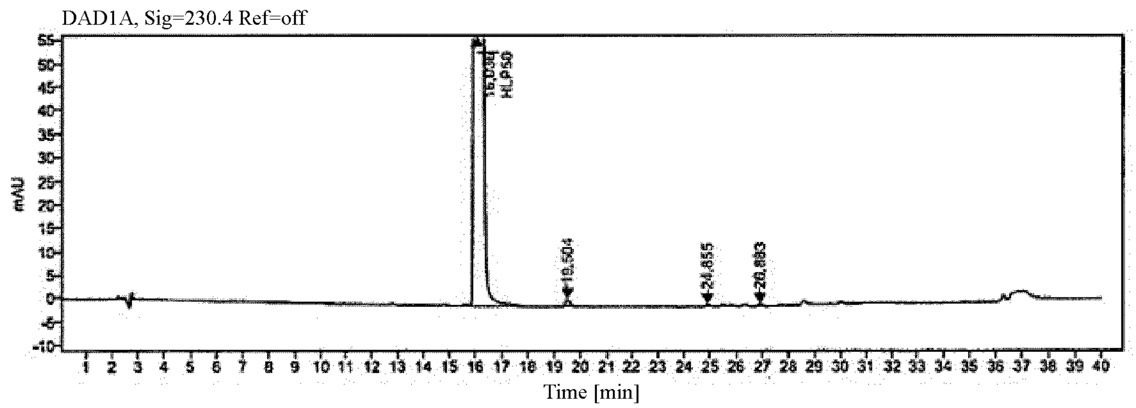


**Signal:** **DAD1A, Sig=230.4 Ref=off**

| RT [min] | Type | Width [min] | Area | Height | Area% | RRT | Name |
|---|---|---|---|---|---|---|---|
| 16.036 | VB | 2.78 | 21510.5703 | 1781.60 | 99.8682 | 1.000 | HLP50 |
| 19.504 | BB | 0.52 | 18.0306 | 1.55 | 0.0837 | 1.216 | |
| 24.855 | BB | 0.50 | 5.1749 | 0.48 | 0.0240 | 1.550 | |
| 26.883 | BBA | 0.57 | 5.1740 | 0.48 | 0.0240 | 1.676 | |
| | | Sum | 21538.95 | | | | |

Fig. 10

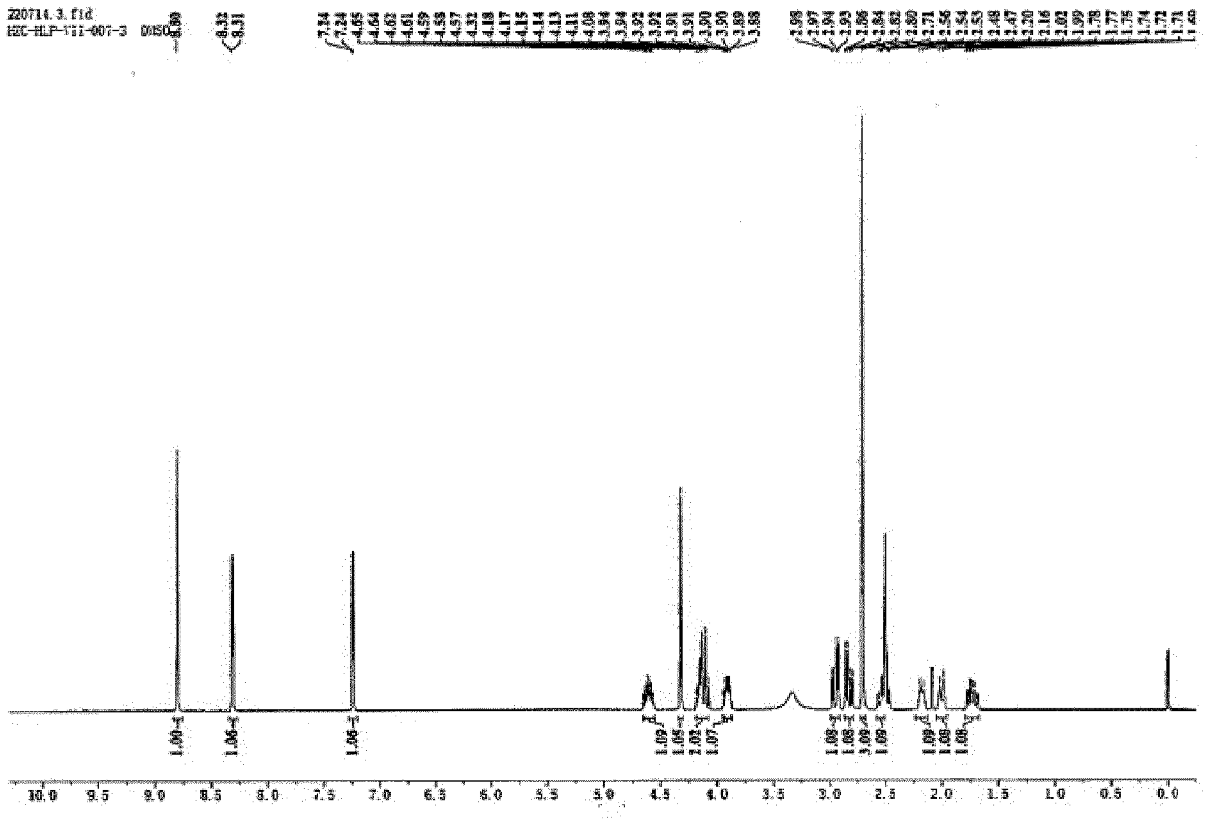

Fig. 11

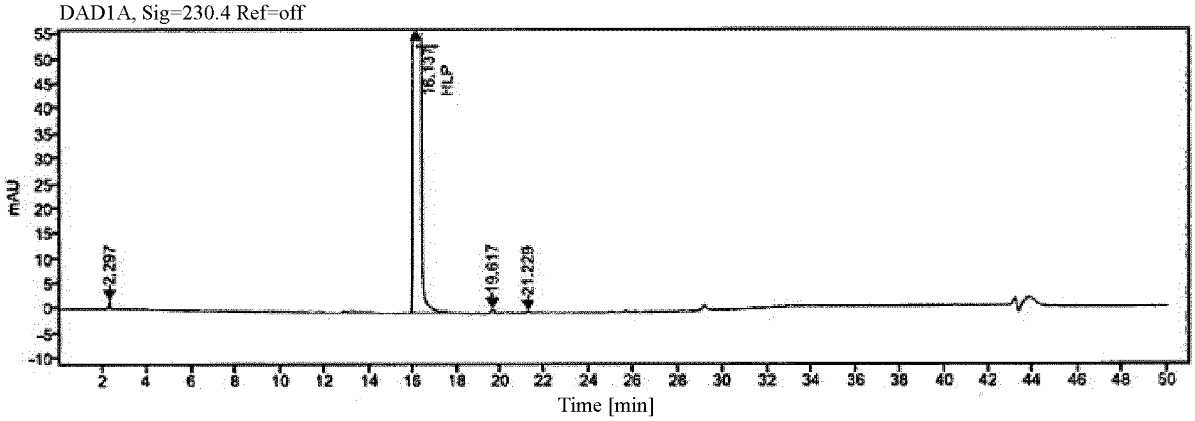


**Signal:** **DAD1A, Sig=230.4 Ref=off**

| RT [min] | Type | Width [min] | Area | Height | Area% | RRT | Name |
|---|---|---|---|---|---|---|---|
| 2.297 | BBA | 0.16 | 6.3457 | 1.48 | 0.0304 | 0.142 | |
| 16.137 | BB | 2.10 | 20881.9962 | 1817.89 | 99.9034 | 1.000 | HLP |
| 19.617 | BB | 0.49 | 9.1486 | 0.85 | 0.0438 | 1.216 | |
| 21.229 | BBA | 0.59 | 4.6974 | 0.38 | 0.0225 | 1.316 | |
| | | Sum | 20902.19 | | | | |

Fig. 12

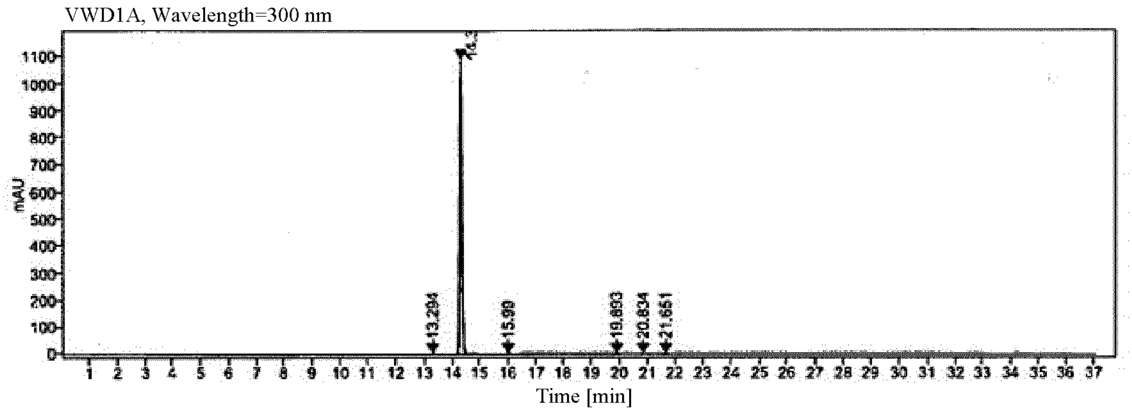


| Signal: | VWD1A, Wavelength=300 nm | | | | | | | |
|---|---|---|---|---|---|---|---|---|
| RT [min] | Width [min] | Area | Height | Area% | R | S/N | T | N |
| 13.294 | 0.26 | 0.37 | 0.7 | 0.006 | | | | |
| 14.300 | 0.79 | 5678.46 | 1088.34 | 99.964 | | | | |
| 15.990 | 0.35 | 0.18 | 0.03 | 0.003 | | | | |
| 19.893 | 0.28 | 0.97 | 0.16 | 0.017 | | | | |
| 20.834 | 0.31 | 0.39 | 0.07 | 0.007 | | | | |
| 21.651 | 0.19 | 0.16 | 0.03 | 0.003 | | | | |
| | Sum | 5680.53 | | | | | | |

Fig. 13

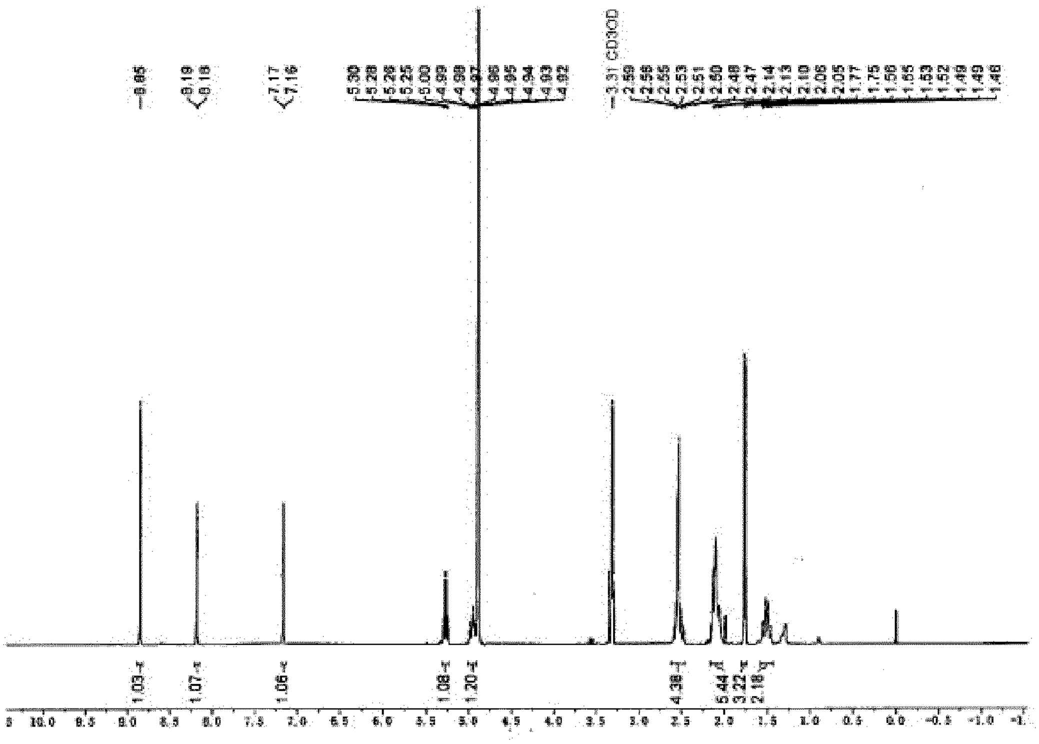

Fig. 14

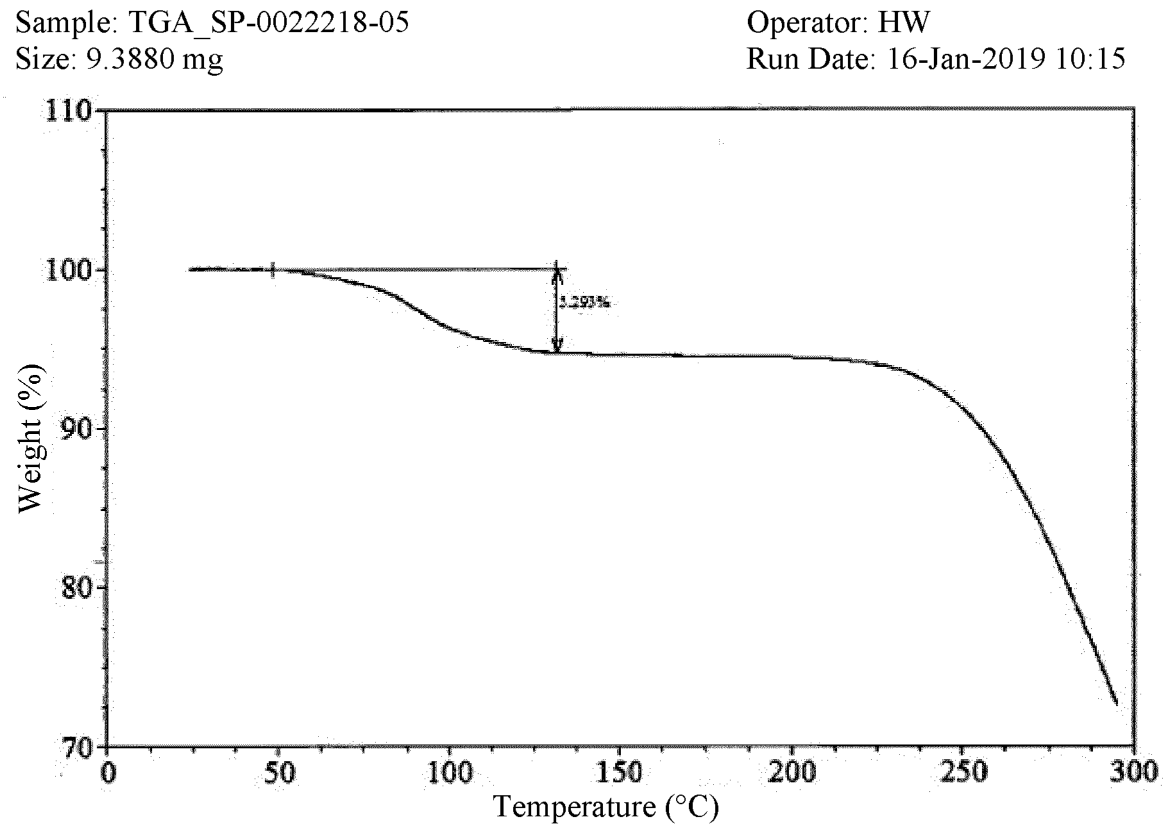
Sample: TGA_SP-0022218-05
Size: 9.3880 mg
Operator: HW
Run Date: 16-Jan-2019 10:15
110
100
90
80
70
Weight (%)
0
50
100
150
200
250
300
Temperature (°C)

Fig. 15

**INTERNATIONAL SEARCH REPORT**

International application No.
**PCT/CN2022/139966**

**A. CLASSIFICATION OF SUBJECT MATTER**

C07D 491/147 (2006.01)i;C07D 491/048(2006.01)i

According to International Patent Classification (IPC) or to both national classification and IPC

**B. FIELDS SEARCHED**

Minimum documentation searched (classification system followed by classification symbols)

IPC: C07D491/-

Documentation searched other than minimum documentation to the extent that such documents are included in the fields searched

Electronic data base consulted during the international search (name of data base and, where practicable, search terms used)

CNABS; CNTXT; VEN; WOTXT; USTXT; EPTXT; GBTXT; CATXT; CNKI; 万方, WANFANG; STN: 呋喃, 咪唑, 吡啶, 吡喃, 氰基, 硝基, 还原, 乳酸, 乙酸, 乙酸酐, 梁从新, 李双江, 黄君珉, 施国强, 段亚亚, 严普查, 陆国彪, 殷宏飞, 金煜东, 廖思敏, 杭州高光制药, 杭州澳赛诺生物, 广州高瓴制药, +furo+, +imidazo+, +pyridin+, +pyran+, +nitrile+, +nitro+, reduc+, lactic acid, acetic acid, acetic anhydride, 化合物3-6的合成

**C. DOCUMENTS CONSIDERED TO BE RELEVANT**

| Category* | Citation of document, with indication, where appropriate, of the relevant passages | Relevant to claim No. |
|---|---|---|
| Y | WO 2020244348 A1 (GUANGZHOU GAOLING PHARMACEUTICALS, LTD.) 10 December 2020 (2020-12-10)<br>pages 3-5, and embodiments 1-29 | 1-43 |
| Y | WO 2020244349 A1 (GUANGZHOU GAOLING PHARMACEUTICALS, LTD.) 10 December 2020 (2020-12-10)<br>pages 3-5, and embodiments 1-29 | 1-43 |
| PY | CN 114213424 A (HANGZHOU ALLSINO BIOTECHNOLOGY CO., LTD.) 22 March 2022 (2022-03-22)<br>claims 1-10, and embodiments 1-2 | 22-29 |
| A | CN 108366994 A (LIANG CONGXIN) 03 August 2018 (2018-08-03)<br>entire document | 1-43 |
| A | CN 106432232 A (SUZHOU HANDE CHUANGHONG BIOCHEMICAL TECHNOLOGY CO., LTD.) 22 February 2017 (2017-02-22)<br>entire document | 1-43 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03.03月2023（03.03.2023）** | **14 March 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/139966**

| C. DOCUMENTS CONSIDERED TO BE RELEVANT | | |
|---|---|---|
| Category* | Citation of document, with indication, where appropriate, of the relevant passages | Relevant to claim No. |
| A | CN 112047938 A (BEIJING TIDE PHARMACEUTICAL CO., LTD.) 08 December 2020 (2020-12-08)<br>entire document | 1-43 |
| Y | Jeremy M. Murray et al. "Potent and Highly Selective Benzimidazole Inhibitors of PI3-Kinase Delta"<br>*Journal of Medicinal Chemistry,* Vol. 55, No. 17, 09 August 2012 (2012-08-09), ISSN: 0022-2623,<br>page 7691, scheme 3 | 1-43 |

Form PCT/ISA/210 (second sheet) (July 2022)

**INTERNATIONAL SEARCH REPORT**
**Information on patent family members**

International application No.
**PCT/CN2022/139966**

| Patent document cited in search report | Publication date (day/month/year) | Patent family member(s) | Publication date (day/month/year) |
|---|---|---|---|
| WO 2020244348 A1 | 10 December 2020 | EP 3981771 A1 | 13 April 2022 |
| | | BR 112021024533 A2 | 18 January 2022 |
| | | US 2022227777 A1 | 21 July 2022 |
| | | AU 2020289149 A1 | 06 January 2022 |
| | | IL 288348 A | 01 January 2022 |
| | | CA 3142625 A1 | 10 December 2020 |
| | | JP 2022543183 A | 11 October 2022 |
| | | KR 20220016955 A | 10 February 2022 |
| WO 2020244349 A1 | 10 December 2020 | EA 202192871 A1 | 22 March 2022 |
| | | AU 2020287139 A1 | 06 January 2022 |
| | | AU 2020287139 A2 | 13 January 2022 |
| | | EP 3981398 A1 | 13 April 2022 |
| | | US 2022242873 A1 | 04 August 2022 |
| | | JP 2022537919 A | 31 August 2022 |
| | | KR 20220017990 A | 14 February 2022 |
| | | BR 112021024530 A2 | 18 January 2022 |
| | | CA 3142629 A1 | 10 December 2020 |
| | | IL 288349 A | 01 January 2022 |
| CN 114213424 A | 22 March 2022 | None | |
| CN 108366994 A | 03 August 2018 | CA 3039178 A1 | 12 April 2018 |
| | | HUE 058120 T2 | 28 July 2022 |
| | | ES 2901216 T3 | 21 March 2022 |
| | | BR 112019005969 A2 | 18 June 2019 |
| | | PT 3509591 T | 27 December 2021 |
| | | IL 291265 A | 01 May 2022 |
| | | IL 291265 B | 01 January 2023 |
| | | US 2019256523 A1 | 22 August 2019 |
| | | US 10738060 B2 | 11 August 2020 |
| | | RS 62695 B1 | 31 January 2022 |
| | | WO 2018067422 A1 | 12 April 2018 |
| | | IL 265358 A | 30 May 2019 |
| | | IL 265358 B | 01 April 2022 |
| | | DK 3509591 T3 | 20 December 2021 |
| | | PL 3509591 T3 | 31 January 2022 |
| | | AU 2022201058 A1 | 10 March 2022 |
| | | JP 2019537559 A | 26 December 2019 |
| | | JP 7089141 B2 | 22 June 2022 |
| | | HRP 20211965 T1 | 18 March 2022 |
| | | NZ 751284 A | 30 September 2022 |
| | | HK 1253040 A1 | 06 June 2019 |
| | | MX 2019003649 A | 14 August 2019 |
| | | EA 201990523 A1 | 31 October 2019 |
| | | EP 3509591 A1 | 17 July 2019 |
| | | EP 3509591 A4 | 25 March 2020 |
| | | EP 3509591 B1 | 17 November 2021 |
| | | KR 20190057069 A | 27 May 2019 |
| | | KR 102399848 B1 | 19 May 2022 |
| | | AU 2017339417 A1 | 28 March 2019 |
| | | AU 2017339417 B2 | 18 November 2021 |
| | | AU 2017339417 C1 | 02 June 2022 |

Form PCT/ISA/210 (patent family annex) (July 2022)

**INTERNATIONAL SEARCH REPORT**
**Information on patent family members**

International application No.
**PCT/CN2022/139966**

| Patent document cited in search report | Publication date (day/month/year) | Patent family member(s) | Publication date (day/month/year) |
|---|---|---|---|
| | | IL 291267 A | 01 May 2022 |
| | | IL 291267 B | 01 January 2023 |
| | | AU 2022201061 A1 | 10 March 2022 |
| CN 106432232 A | 22 February 2017 | None | |
| CN 112047938 A | 08 December 2020 | JP 2022536313 A | 15 August 2022 |
| | | WO 2020244613 A1 | 10 December 2020 |
| | | EP 3967694 A1 | 16 March 2022 |
| | | EP 3967694 A4 | 07 December 2022 |
| | | KR 20220016225 A | 08 February 2022 |
| | | US 2022378799 A1 | 01 December 2022 |
| | | TW 202112764 A | 01 April 2021 |
| | | TWI 778366 B | 21 September 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018067422 A1
- WO 2020244348 A1
- WO 2020244349 A1